# EUROPÄISCHE PATENTSCHRIFT

(11) **EP 2 175 785 B1**
(45) Veröffentlichungstag und Bekanntmachung des Hinweises auf die Patenterteilung: **13.04.2011**
(21) Anmeldenummer: 08715957.0
(22) Anmeldetag: 22.02.2008
(51) Int. Cl.: A61B 17/32, A61B 10/02

(54) **VORRICHTUNG ZUM AUSSCHNEIDEN UND ENTFERNEN VON GEWEBEZYLINDERN AUS EINEM GEWEBE UND DEREN VERWENDUNG**
DEVICE FOR CUTTING OUT AND REMOVING CYLINDERS OF TISSUE FROM A TISSUE AND THE USE THEREOF
PROCÉDÉS POUR TRAITEMENT CONTINU DE RÉSINE DE POLYTÉTRAFLUOROÉTHYLÈNE (PTFE)

(30) Priorität: 22.02.2007 DE 102007008751; 17.10.2007 DE 102007049796
(43) Veröffentlichungstag der Anmeldung: 21.04.2010
(73) Patentinhaber: WISAP Gesellschaft für wissenschaftlichen Apparatebau mbH, 82054 Sauerlach (DE)
(72) Erfinder: REPPENTHIEN, Joachim, 82054 Sauerlach (DE)
(74) Vertreter: Grape, Knut
(86) Internationale Anmeldenummer: PCT/EP2008/001406
(87) Internationale Veröffentlichungsnummer: WO 2008/101712

(56) Entgegenhaltungen:
- EP-A- 0 522 125
- EP-A- 1 348 386
- EP-A- 1 681 022
- WO-A-00/19909
- WO-A-01/91653
- WO-A-98/53865
- WO-A-2005/104966
- DE-A1- 4 440 035
- DE-A1- 10 202 885
- US-A- 4 306 570
- US-A- 4 840 184
- US-A- 5 242 412
- US-A- 5 584 850
- US-A- 5 779 697
- US-A- 5 916 198
- US-B1- 6 439 541
- US-B1- 6 468 228

## Beschreibung

Die Erfindung betrifft eine Vorrichtung zum Ausschneiden und Entfernen von Gewebezylindern aus einem Gewebe, das sich innerhalb einer Körper- oder Gelenkhöhle und/oder in bzw. an einem Wandbereich davon befindet.

Derartige Vorrichtungen sind allgemein bekannt. Allerdings haben sich derartige Vorrichtungen in der Praxis als ausgesprochen nachteilig erwiesen. So dienen solche Vorrichtungen unter gleichzeitiger Verwendung eines Trokars und einer Trokarhülse beispielsweise auf dem medizinischen Gebiet der Gynäkologie einem Entfernen von Gewebe aus einer Gebärmutter, einer Gebärmutter insgesamt oder zur Behandlung von Myomen, etc. Aus der EP 0 522 125 B1 ist zum Beispiel eine derartige Vorrichtung bekannt. Derartige Vorrichtungen finden darüber hinaus in der Medizin auf zahlreichen und zum Teil unterschiedlichsten weiteren Fachgebieten Verwendung, etwa bei operativen Eingriffen im Bauchraum, am Magen und zur Gallenblasenentfernung oder Blinddarmentfernung. Von Nachteil bei sämtlichen dieser Vorrichtung ist deren konstruktive Ausbildung insgesamt. Zumeist besitzen derartige Vorrichtungen eine verhältnismäßig aufwendige und relativ wenig kompakte sowie stabile Bauweise. Besonders nachteilig wirkt sich bei allen derartigen Vorrichtungen aus, dass diese Vorrichtungen nach Ausschneiden bzw. Ausstanzen eines Gewebezylinders zusammen mit einem weiteren gesonderten Instrument, welches heutzutage unerlässlich zum Einsatz kommt, insbesondere einem Greif- oder sonstigen Schneideinstrument, wie einem Krallengeifer oder einem Myombohrer etc., zum Entfernen des Gewebezylinders aus dem Operationsgebiet durch die Trokarhülse herausgezogen werden müssen. Damit einhergehend gestaltet sich die Handhabung derartiger Vorrichtungen insgesamt ausgesprochen aufwendig, für den Patienten wenig schonend und zeitintensiv. Schließlich ergeben sich aus dem ständigen Einführen und anschließenden Herausziehen derartiger Vorrichtungen in die bzw. aus der Trokarhülse ein erhöhtes Risiko für die Patienten infolge von zum Beispiel einer Traumatisierung des umliegenden Gewebes bei der Aushülsung und ähnlichem.

EP 1681022 offenbart eine Vorrichtung zum Ausschneiden und Entfernen von Gewebezylindern aus einem Gewebe gemäß dem Oberbegriff des Anspruchs 1. Der Gegenstand des Anspruchs 1 unterscheidet sich von der EP1681022 wenigstens dadurch, dass ein kreisförmiger, umlaufender Steg und eine kreisförmige, umlaufende Ausnehmung, wie in dem Kennzeichen des Anspruchs 1 definiert, vorgesehen sind. Auf diese Weise wird die Zentrierung der Dichtungselemente verbessert, was weiterhin einer zusätzlichen Sicherung und einer erhöhten Dichtungswirkung dient.

US 5,916,198; DE 44 40 035 A1; US 6,468,228 B1 und WO 2005/104966 A1 offenbaren Vorrichtungen zum Ausschneiden und Entfernen von Gewebezylindern aus einem Gewebe, die ein Dichtungsmittel bzw. eine Dichtungsvorrichtung zeigen, wobei eine Merkmalkombination aus einem Steg und einer Ausnehmung, wie im Kennzechen des Anspruchs 1 definiert ist, nicht vorhanden ist.

US 4,306,570 offenbart eine Biopsienadel, bei welcher zwei Rohre mit an deren distalen Enden angebrachten Schneiden über ein Getriebe zum gegenläufigen Drehen ausgebildet sind. Eine Dichtungseinrichtung ist nicht vorhanden.

Die Ausgestaltung der erfindungsgemäßen Vorrichtung zum Ausschneiden und Entfernen von Gewebezylindern aus einem Gewebe, das sich innerhalb einer Körper- oder Gelenkhöhle und/oder in bzw. an einem Wandbereich davon befindet, die eine Schneideeinrichtung mit einem hohlzylindrischen Grundkörper einer distalen Öffnung an einem distalen Ende des Grundkörpers und einem die distale Öffnung umgebenden Schneideelement, eine Betätigungseinrichtung zum Drehen des Grundkörpers um dessen Längsachse an einem proximalen Ende und/oder an einem dem proximalen Ende zugewandten Bereich des Grundkörpers und eine Dichtungseinrichtung zum Verschließen und Freigeben einer proximalen Öffnung an dem proximalen Ende des Grundkörpers umfasst weist, eine einfache, kompakte und stabile Bauweise der gesamten Vorrichtung auf. Weiterhin hat die erfindungsgemäße Vorrichtung, die auch als Morcellator bezeichnet werden kann, den wesentlichen Vorteil, dass die Vorrichtung nach Ausschneiden bzw. Ausstanzen eines Gewebezylinders nicht zusammen mit einem weiteren Instrument, insbesondere einem Greif- oder sonstigen Schneideinstrument, wie einem Krallengeifer oder einem Myombohrer etc., zum Entfernen des Gewebezylinders aus dem Operationsgebiet durch die Trokarhülse herausgezogen werden muss. Vielmehr ermöglicht die erfindungsgemäße Vorrichtung dem Anwender, morcelliertes Gewebe zu extrahieren und die Vorrichtung selbst da-bei zugleich in bzw. in dem Operationsgebiet zu belassen. Nicht zuletzt hieraus resultierend ergibt sich eine ausgesprochen leichte, sehr schonende und vor allem zeitsparende Handhabung der Vorrichtung insgesamt. Schließlich lässt sich mit der Vorrichtung nach der Erfindung eine erheblich verbesserte und erhöhte Patientensicherheit erreichen.

Weitere vorteilhafte Einzelheiten der erfindungsgemäßen Vorrichtung sind in den Ansprüchen 2 bis 17 beschrieben.

Die konstruktiven Maßnahmen des Anspruchs 2 dienen in ganz vorteilhafter Weise einer einfachen, schnellen und zuverlässigen sowie vor allem kostengünstigen Herstellung und Montage. Danach sind das Gehäuseelement und das Verschlusselement des Gehäuses über eine Gewindeverbindung oder eine Rast- oder Schnappverbindung miteinander lösbar oder unlösbar verbunden. Die konstruktive Ausgestaltung, eine lösbare oder eine unlösbare Verbindung zwischen Gehäuseelement und Verschlusselement des Gehäuses vorzusehen, richtet sich dabei wesentlich nach der Verwendungs- und Bestimmungsart der erfindungsgemäßen Vorrichtung, d.h. ob die Vorrichtung zum Einmal- oder Mehrfach-Gebrauch vorgesehen sein soll. Dies wiederum, wie auch umgekehrt, wirkt sich erheblich auf die Ausbildung der Vorrichtung insgesamt bzw. die Auswahl der Materialien, aus welchen die einzelnen Komponenten der erfindungsgemäßen Vorrichtung bestehen, aus.

Ist die Vorrichtung beispielsweise lediglich für einen Einmal-Gebrauch bestimmt, wird also nach Gebrauch vollständig entsorgt, können das Gehäuseelement und das Verschlusselement des Gehäuses nach Anspruch 3 vorzugsweise über ein Gewinde unter Zwischenanordnung eines Klebemittels, insbesondere eines Silikonklebers, miteinander unlösbar verbunden sein. Für den Mehrfach-Gebrauch werden das Gehäuseelement und das Verschlusselement des Gehäuses, die dann sicherlich auch aus Metall ausgebildet sind, hingegen mittels Gewinde miteinander lediglich verschraubt.

Alternativ zur Gewindeverbindung kann gleichermaßen eine Rast- oder Schnappverbindung vorgesehen sein, bei welcher das Gehäuseelement und das Verschlusselement des Gehäuses nach Anspruch 4 über wenigstens einen Rastvorsprung oder dergleichen Rastnase an dem Gehäuseelement und mindestens einen weiteren Rastvorsprung oder dergleichen Rastnase an dem Verschlusselement, die zusammenwirken und in gegenseitigen Eingriff bringbar sind, miteinander verbunden sind. Eine kinematische Umkehr dessen, den wenigstens einen Rastvorsprung oder dergleichen Rastnase an dem Verschlusselement und die mindestens eine Rastausnehmung oder dergleichen an dem Gehäuseelement vorzusehen, ist ebenso möglich. Anstelle des mindestens einen weiteren Rastvorsprunges kann ebenso mindestens eine Rastausnehmung oder dergleichen vorgesehen sein, der mit dem wenigstens einen Rastvorsprung zusammenwirkt.

Für einen Einmal-Gebrauch ist die Vorrichtung nach der Erfindung in diesem Zusammenhang vorteilhafterweise entsprechend den Merkmalen des Anspruchs 5 ausgestaltet. Demnach ist/sind der wenigstens eine Rastvorsprung oder dergleichen Rastnase an dem Gehäuseelement bzw. an dem Verschlusselement und der mindestens eine weitere Rastvorsprung oder dergleichen Rastnase an dem Verschlusselement bzw. an dem Gehäuseelement derart ausgebildet, dass das Gehäuseelement und das Verschlusselement des Gehäuses entweder einerseits nach Ineingriffbringung von dem wenigstens einen Rastvorsprung oder dergleichen Rastnase und dem mindestens einen weiteren Rastvorsprung oder dergleichen Rastnase miteinander unlösbar verbunden sind oder andererseits nach anschließender Außereingriffbringung von dem wenigstens einen Rastvorsprung oder dergleichen Rastnase und dem mindestens einen weiteren Rastvorsprung oder dergleichen Rastnase miteinander unverbindbar sind. Bei letzterer Ausgestaltung wäre zum Beispiel denkbar, einen der beiden Rastvorsprünge oder dergleichen Rastnasen mit einer Sollbruchstelle zu versehen, so dass dieser Rastvorsprung oder dergleichen Rastnase bei Beaufschlagung einer bestimmten Zugkraft von dem Gehäuseelement bzw. von dem Verschlusselement abreißt. Anstelle des mindestens einen weiteren Rastvorsprunges kann ebenso mindestens eine Rastausnehmung oder dergleichen vorgesehen sein, der mit dem wenigstens einen Rastvorsprung zusammenwirkt.

Dabei ist erfindungsgemäß vorgesehen, dass das eine der zwei Dichtungselemente der Dichtungseinrichtung nach Anspruch 6 eine Schlitzöffnung, die in einer Ebene, welche gegenüber einer durch die Grundfläche des Dichtungselementes aufgespannten Ebene in Richtung des distalen Endes des Grundkörpers der Schneideeinrichtung versetzt angeordnet ist, umfasst. So wird das eine der zwei Dichtungselemente aufgrund seiner Formgebung und Anordnung in dem Gehäuse in entgegengesetzter Richtung zu der Wirkung eines innerhalb einer Körper- oder Gelenkhöhle künstlich aufgebauten Überdruckes, zum Beispiel durch CO₂-Insufflation, in dessen Schließstellung gezwungen und gehalten. Dabei wird das Dichtungselement an ein weiteres Instrument bzw. dessen Schaft, zum Beispiel ein Greif- oder sonstiges Schneideinstrument, insbesondere ein Krallengeifer oder ein Myombohrer, außenseitig unter vollständiger Abdichtung angedrückt. Demnach ist sichergestellt, dass der Überdruck, welcher zum Operieren, d.h. Ausschneiden bzw. Ausstanzen eines Gewebezylinders aus einem Gewebe und beim anschließenden Entfernen des ausgeschnittenen bzw. ausgestanzten Gewebezylinders aus dem Operationsgebiet erforderlich ist, in jedem Fall aufrechterhalten bleibt. Mithin ist ein Entweichen von Gas, wie CO₂ oder dergleichen, aus der Körper- oder Gelenkhöhle des Operationsgebietes ausgeschlossen und damit einer Behinderung des Aushülsungsvorganges oder einer Traumatisierung des umliegenden Gewebes bei der Aushülsung entgegenwirkt.

Insbesondere liegt es im Rahmen der Erfindung, dass das eine der zwei Dichtungselemente nach Anspruch 7 wenigstens zwei Dichtlippen aufweist, die zum Verschließen und Freigeben der Schlitzöffnung in gegenseitigem Wirkeingriff stehen.

Darüber hinaus weist das andere der zwei Dichtungselemente der Dichtungseinrichtung nach Anspruch 8 eine mittige, etwa kreisförmige Ausnehmung zum Hindurchführen eines weiteren Instrumentes, insbesondere eines Greif- oder sonstiges Schneideinstrumentes, vorzugsweise eines Krallengeifers oder ein Myombohrers, auf.

Die mittige, etwa kreisförmige Ausnehmung des anderen der zwei Dichtungselemente ist nach Anspruch 9 vorzugsweise mit einem Innendurchmesser ausgebildet, welcher kleiner oder gleich einem Außendurchmesser eines weiteren, durch die Ausnehmung hindurchzuführenden Instrumentes, insbesondere eines Greif- oder sonstiges Schneideinstrumentes, vorzugsweise eines Krallengeifers oder ein Myombohrers, ist.

Des Weiteren liegt es im Rahmen der Erfindung, dass das andere der zwei Dichtungselemente entsprechend konstruktiver Ausgestaltung nach Anspruch 10 in dem Gehäuse auf der Längsachse des Grundkörpers der Schneideeinrichtung zwischen dem proximalen Ende des Grundkörpers und dem einen der zwei Dichtungselemente, insbesondere der durch die Grundfläche des einen der zwei Dichtungselemente aufgespannten Ebene benachbart, angeordnet ist. Auf diese Weise wird ein gegenseitiges Zusammenwirken der beiden Dichtungselemente erreicht, wodurch die Abdichtung noch zusätzlich verbessert werden kann.

Entsprechend Anspruch 11 können der kreisförmige, umlaufende Steg und/oder die kreisförmige, umlaufende Ausnehmung des einen und/oder des anderen der zwei Dichtungselemente der Dichtungseinrichtung vorzugsweise im Querschnitt viereckig, insbesondere quadratisch oder rechteckförmig, U-förmig oder V-förmig ausgebildet ist/sind. Der Vorteil, wonach ein gegenseitiges Zusammenwirken der beiden Dichtungselemente erreicht und dadurch die Abdichtung noch zusätzlich verbessert werden kann, wird bei der erfindungsgemäßen Vorrichtung weiter dadurch unterstützt, dass die zwei Dichtungselemente der Dichtungseinrichtung nach Anspruch 12 durch eine Klemmscheibe oder dergleichen in dem Gehäuse zueinander fixierbar sind.

Zur Verbesserung der Dichtungswirkung ist/sind das wenigstens eine, insbesondere die zwei, Dichtungselement/e der Dichtungseinrichtung nach Anspruch 13 aus flexiblen Kunststoff, insbesondere aus Silikon, gebildet.

Von ausgesprochen großer Bedeutung für die erfindungsgemäße Vorrichtung sind des Weiteren die Merkmale des Anspruchs 14, wonach die Dichtungseinrichtung zum Verschließen und Freigeben der proximalen Öffnung an dem proximalen Ende des Grundkörpers zum Ausschneiden und Entfernen von Gewebezylindern aus einem Gewebe an dem hohlzylindrischen Grundkörper der Schneideeinrichtung angeordnet ist, das von wenigstens einer weiteren Schneideeinrichtung umgeben ist, die ineinander angeordnet und zueinander relativ sowie jeweils einzeln drehbar ausgebildet sind, wobei die Betätigungseinrichtung mindestens eine Antriebseinrichtung zum Drehen des jeweiligen Grundkörpers der wenigstens zwei Schneideeinrichtungen um dessen Längsachse umfasst.

Darüber hinaus dienen die Merkmale der Ansprüche 15 bis 17 einer besonders effizienten und individuell anpassbaren Handhabung und Betriebsweise der erfindungsgemäßen Vorrichtung während einer Operation. Damit einhergehend lässt sich ein operativer Eingriff für einen Patienten erheblich schonender ausführen. Die Vielseitigkeit der Vorrichtung wird dadurch zusätzlich verbessert.

Von besonderem Interesse ist das Schneideelement-der Scheideeinrichtung, welches die distale Öffnung umgibt, nach Auspruch 18 im wesentlichen angefast, kegelförmig, konisch oder schräg von der distalen Öffnung abgewandt nach außen verlaufend ausgebildet ist. Die Schneide weist einen sehr scharfen Wellenschliff auf, der bei einer Drehbewegung des Grundkörpers eine exakte Schnittführung in dem Gewebe bewirkt, ohne dass das verbleibende Gewebe zu stark traumatisiert wird. So lässt sich das Gewebe aushülsen, ohne dass umliegendes Gewebe bei dem Vorgang deformiert wird.

In diesem Zusammenhang weist das Schneideelement der Schneideeinrichtung vorteilhafterweise einen umlaufenden bzw. kontinuierlichen oder diskontinuierlichen Glattschliff, Wellenschliff oder Zahnschliff auf.

Das Schneideelement der Schneideeinrichtung ist an dem vordersten Rand der distalen Öffnung bevorzugt mit einem Wellenschliff ausgestattet. Bei dieser Ausgestaltung der Erfindung ergeben sich mehrere, über den Umfang verteilte Kreisbogenabschnitte mit axial zurücktretenden Wellentälern und axial hervortretenden Wellenbergen. Beispielsweise würden bei vier Wellenvorsprüngen und vier dazwischen liegenden, leicht axial in der Umfangsfläche zurückgeführten Wellentälern Kreisbogenabschnitte etwa im Bereich von 40 bis 45° entstehen. Der Wellenschliff kann dabei umlaufend bzw. kontinuierlich, d.h. über den gesamten Umfang der distalen Öffnung, vorhanden sein. Zweckmäßigerweise kann die Schärfungszone im Übergangsbereich zwischen dem Wellental und dem Wellenvorsprung besonders scharf gestaltet sein, so dass zunächst eine axiale Fixierung des Gewebes und erst anschließend durch die Rotationsbewegung des Grundkörpers das Morcellieren von Gewebezylindern bzw. Gewebehülsen durchgeführt wird. Der Wellenschliff kann gegebenenfalls allerdings auch diskontinuierlich in Art einer schuppenförmigen Anordnung um den Mantelumfang an der distalen Öffnung vorgesehen sein.

Gleiches gilt für die alternative Ausgestaltung der Erfindung. Demnach weist das Schneideelement der Schneideeinrichtung mit dem Zahnschliff über den Umfang verteilte Zähne mit axial zurücktretenden Zahngründen und axial hervortretenden Zahnspitzen auf. In Abhängigkeit von der gewünschten Verwendung ist ein umlaufender bzw. kontinuierlicher oder ein diskontinuierlicher Zahnschliff denkbar.

Einer wesentlichen Vereinfachung von Herstellung und Montage der erfindungsgemäßen Vorrichtung und einer damit einhergehenden Einsparung von Kosten dienen die Maßnahmen, wonach die Betätigungseinrichtung und die Dichtungseinrichtung integral ausgebildet sind.

Dabei ist das Gehäuse der Dichtungseinrichtung, insbesondere das Verschlusselement des Gehäuses, entsprechend den technischen Merkmalen an dessen Umfang mit einem Profil zum manuellen Betätigen und Drehen des Grundkörpers versehen.

In alternativer und/oder kumulativer Ausgestaltung zur Handbetätigung des Grundkörpers beim Ausstanzen oder Aushülsen von Gewebe besteht auch die Möglichkeit, die Rotationsbewegung auf den Grundkörper über einen Motor, der elektrisch, pneumatisch oder hydraulisch angetrieben ist, aufzubringen. Hierzu kann das Gehäuse der Dichtungseinrichtung an dessen Umfang mit einem Profil, insbesondere Zahnrädern, einem Zahnkranz, einem Zahnring oder dergleichen, versehen sein, um mit einem korrespondierenden Gegenprofil eines Motors oder einer Bauteilkomponente davon, beispielsweise einer Getriebeeinheit, zum automatischen Betätigen und Drehen des Grundkörpers zusammenzuwirken.

Das Gehäuse der Dichtungseinrichtung ist an dessen Umfang mit radial angeordneten Bohrungen oder Ausnehmungen bzw. Vorsprüngen versehen, die mit korrespondierend ausgebildeten Vorsprüngen bzw. Bohrungen oder Ausnehmungen an dem Motor oder einer Bauteilkomponente davon, beispielsweise einer Getriebeeinheit, zusammenwirken.

Zweckmäßigerweise ist der Grundkörper mit wenigstens einem Zentrier- und/oder Führungselement versehen, das von der Dichtungseinrichtung beabstandet angeordnet ist und einen Außendurchmesser, welcher größer ist als Außendurchmesser des Grundkörpers, aufweist. Der Außendurchmesser des wenigstens einen Zentrier- und/oder Führungselement ist dem Innendurchmesser einer hohlzylindrischen Trokarhülse eines Trokars angepasst und dient zur Zentrierung und Führung des Grundkörpers. Durch dieses Zentrier- und/oder Führungselement wird demnach eine koaxiale Ausrichtung des Grundkörpers der Schneideeinrichtung bezüglich der Trokarhülse erreicht.

In besonders vorteilhafter Weise kann/können das Gehäuseelement und/oder das Verschlusselement des Gehäuses und/oder das Zentrier- und/oder Führungselement und/oder die Klemmscheibe aus Kunststoff, insbesondere Polyoximethylen, Polyester, ABS, Acryl, Polycarbonat, Tetrafluorethylen oder Impax, duroplastischen Elastomeren, mit oder ohne Glasfaserverstärkung gebildet sein. Die erfindungsgemäße Vorrichtung, die in diesem Fall zum Einmal-Gebrauch vorgesehen ist, lässt sich hierdurch ausgesprochen kostengünstig herstellen.

Alternativ dazu liegt es ebenso im Rahmen der Erfindung, dass der Grundkörper der Schneideeinrichtung und/oder das Gehäuseelement und/oder das Verschlusselement des Gehäuses und/oder das Zentrier- und/oder Führungselement und/oder die Klemmscheibe aus Metall, insbesondere (nicht-rostendem) Stahl, Edelstahl, Aluminium, Messing, Zink, Rotgusslegierungen oder einer Legierung hieraus gebildet ist/sind. In diesem Fall ist die Vorrichtung nach entsprechender thermischer und/oder chemischer Sterilisation für den Mehrfach-Gebrauch wiederverwendbar.

Die Vorrichtung kann zum Ausschneiden und Entfernen von Gewebezylindern aus einem Gewebe, das sich innerhalb einer Körper- oder Gelenkhöhle und/oder in bzw. an einem Wandbereich davon befindet, beispielsweise eines von einem organischen Hohlraum eines menschlichen Körpers wenigstens teilweise aufgenommenen und/oder umgebenen, organischen Körpers, vorzugsweise von Embryonen, Myomen, Geschwülsten, Geschwüren und Karzinomen verwendet werden. Besonders eignet sich die erfindungsgemäße Vorrichtung für laparaskopische, thorakoskopische oder athroskopische und dergleichen minimal-invasive chirurgische Eingriffe. Ohne im Einzelnen dargestellt zu sein, ist es ebenso möglich, mit Hilfe der erfindungsgemäßen Vorrichtung anorganische Körper, wie etwa Gallen- und Blasensteinen oder dergleichen, die sich innerhalb einer Körper- oder Gelenkhöhle und/oder in bzw. an einem Wandbereich davon befindet, in Form von derartigen Körperzylindern auszuschneiden und zu entfernen.

Weitere Merkmale, Vorteile und Einzelheiten der Erfindung ergeben sich aus der nachfolgenden Beschreibung einiger bevorzugter Ausführungsformen der Erfindung sowie anhand der Zeichnungen. Hierbei zeigen:

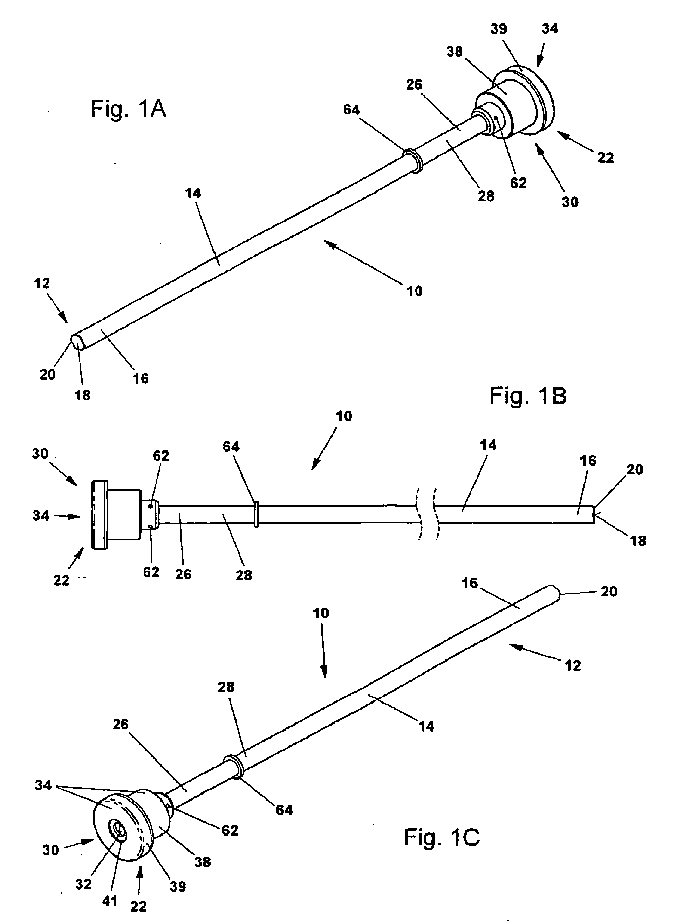

- Fig. 1A, 1B und 1C: eine perspektivische Vorderansicht, eine abgebrochene Seitenansicht und eine perspektivische Rückansicht einer Ausführungsform einer erfindungsgemä- β en Vorrichtung zum Ausschneiden und Entfernen von Ge- webezylindern aus einem Gewebe,

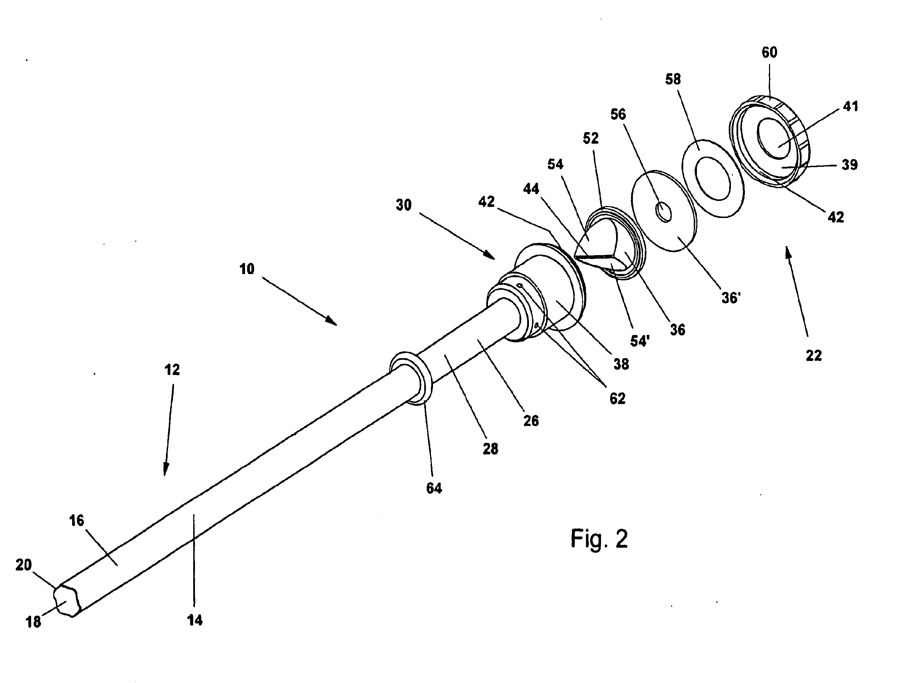

- Fig. 2: eine auseinandergezogene perspektivische Ansicht der Ausführungsform der erfindungsgemäß ausgebildeten Vor- richtung entsprechend den Fig. 1A, in vergrößerter Dar- stellung,

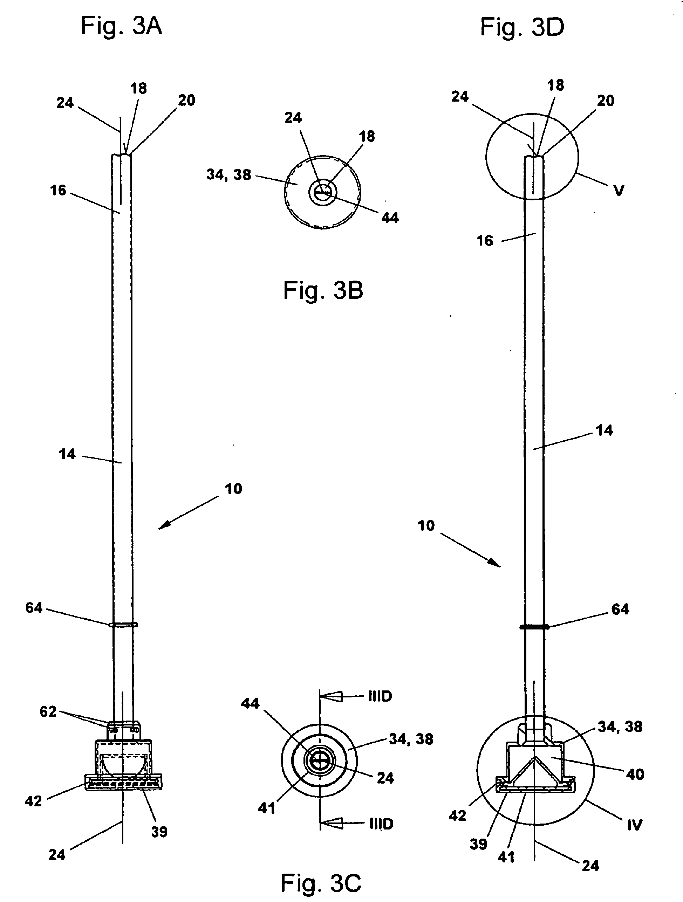

- Fig. 3A bis 3D: eine teilweise aufgebrochene, schematische Seitenansicht, eine Vorderansicht und eine Rück- ansicht der Ausführungsform der erfindungsgemäß ausge- bildeten Vorrichtung entsprechend den Fig. 1A bis 2 so- wie eine Querschnittansicht durch die erfindungsgemäße Vorrichtung entsprechend der Linie IIID-IIID in der Fig. 3C,

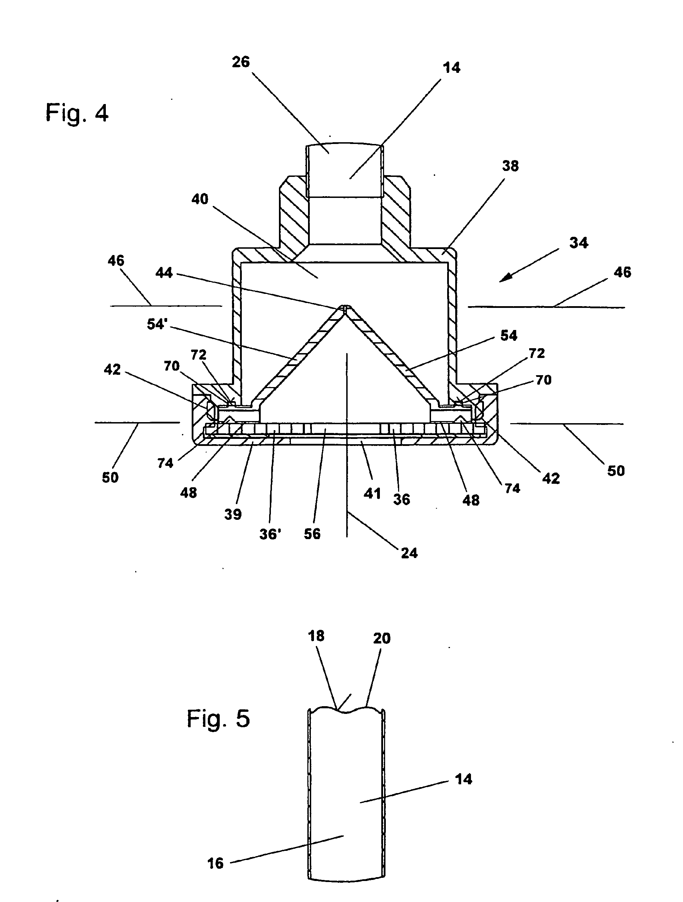

- Fig. 4: eine Querschnittansicht durch eine Ausführungsform ei- ner erfindungsgemäß ausgebildeten Dichtungseinrichtung an dem proximalen Ende der Vorrichtung entsprechend Ausschnitt IV in der Fig. 3D, in vergrößerter Darstel- lung,
- Fig. 5: eine Querschnittansicht durch eine Ausführungsform ei- nes erfindungsgemäß ausgebildeten Schneideelementes an dem distalen Ende der Vorrichtung entsprechend Aus- schnitt V in der Fig. 3D, in vergrößerter Darstellung,

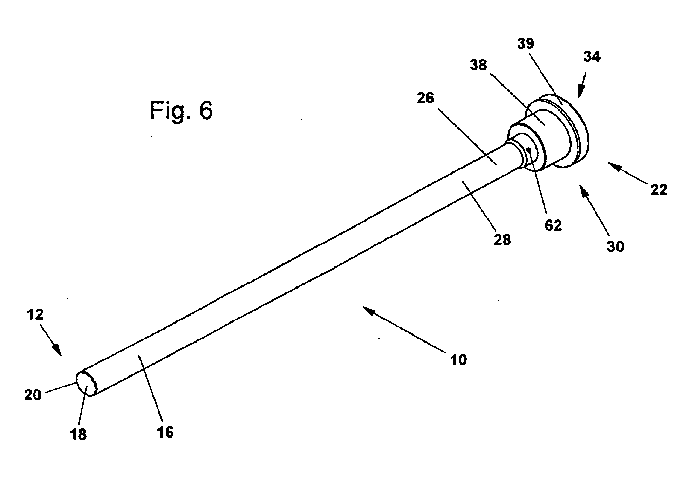

- Fig. 6: eine perspektivische Vorderansicht einer weiteren Aus- führungsform einer erfindungsgemäßen Vorrichtung zum Ausschneiden und Entfernen von Gewebezylindern aus ei- nem Gewebe entsprechend der Fig. 1A,

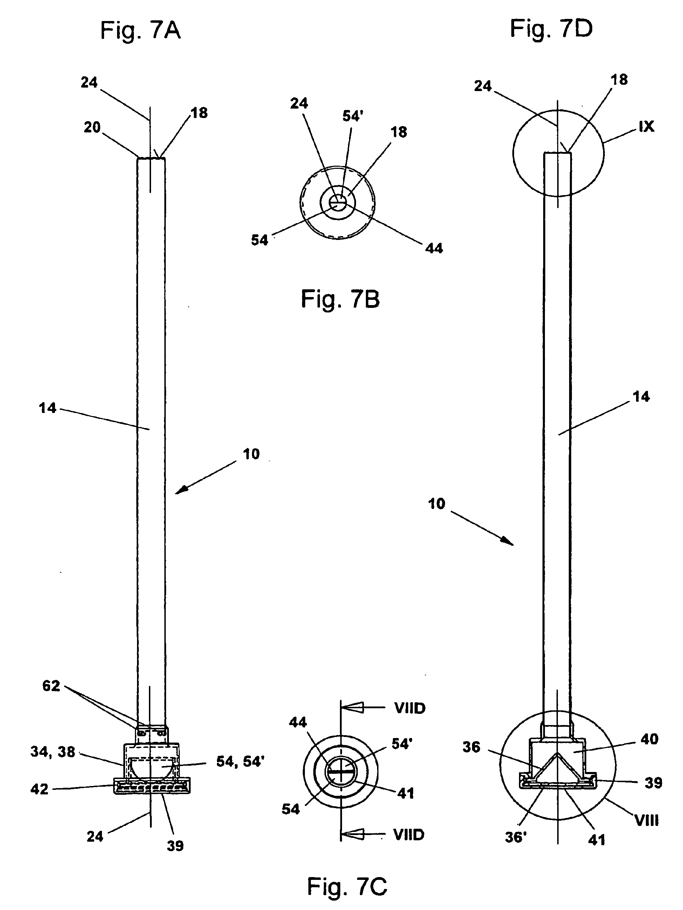

- Fig. 7A bis 7D: eine teilweise aufgebrochene, schematische Seitenansicht, eine Vorderansicht und eine Rückansicht der Ausführungsform der erfindungsgemäß ausgebildeten Vorrichtung entsprechend den Fig. 6 sowie eine Quer- schnittansicht durch die erfindungsgemäße Vorrichtung entsprechend der Linie VIID-VIID in der Fig. 7C,

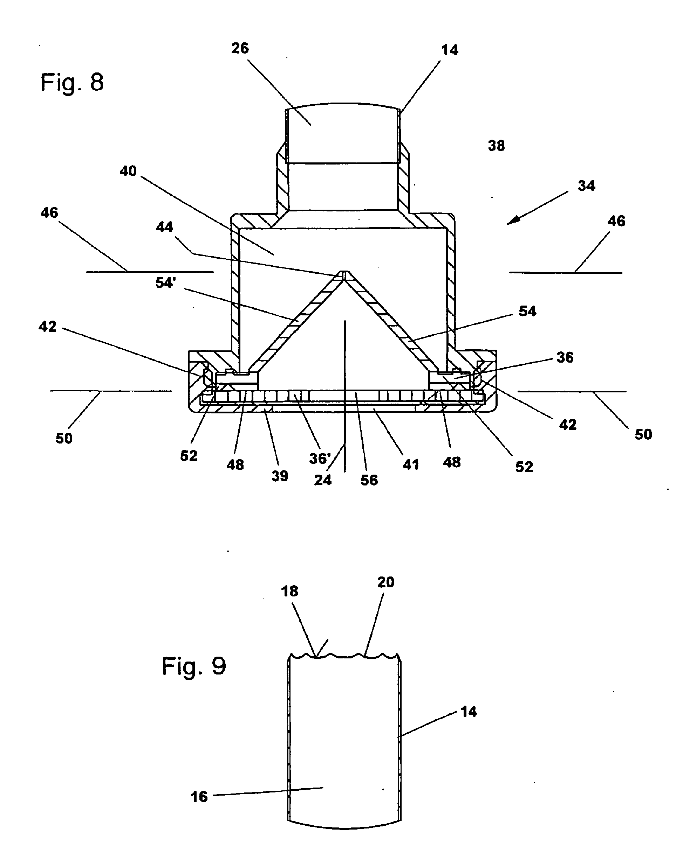

- Fig. 8: eine Querschnittansicht durch eine Ausführungsform einer erfindungsgemäß ausgebildeten Dichtungseinrich- tung an dem proximalen Ende der Vorrichtung entspre- chend Ausschnitt VIII in der Fig. 7D, in vergrößerter Darstellung,
- Fig. 9: eine Querschnittansicht durch eine Ausführungsform eines erfindungsgemäß ausgebildeten Schneideelementes an dem distalen Ende der Vorrichtung entsprechend Aus- schnitt IX in der Fig. 7D, in vergrößerter Darstellung,

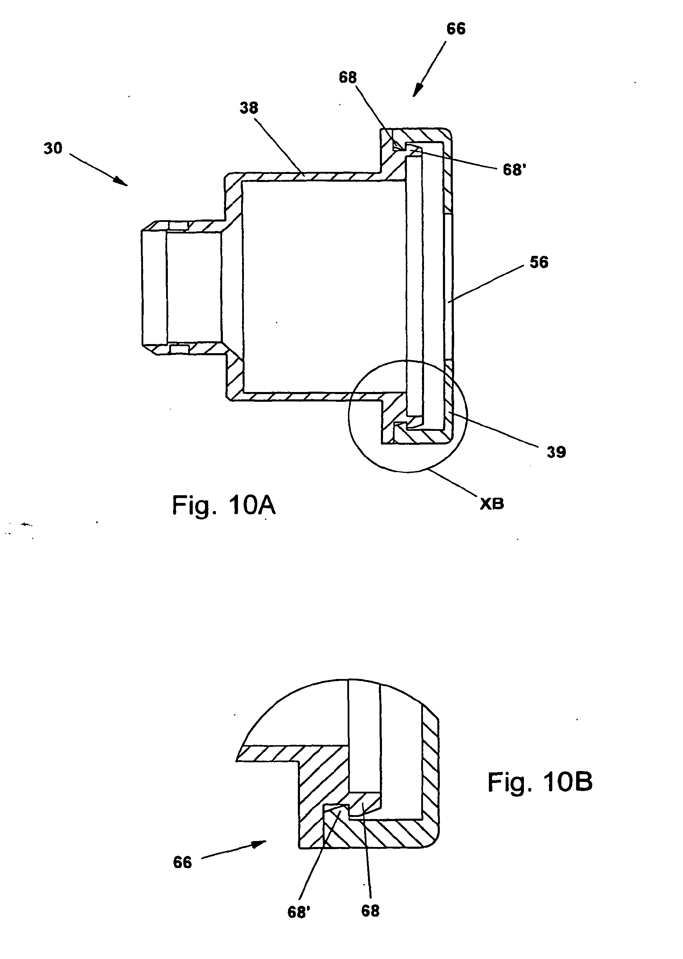

- Fig. 10A und 10B: eine Querschnittansicht durch eine weite- re Ausführungsform einer erfindungsgemäß ausgebildeten Dichtungseinrichtung an dem proximalen Ende der Vor- richtung entsprechend den Fig. 4 und 8 und eine Quer- schnittsansicht entsprechend Ausschnitt XB in der Fig. 10A, in vergrößerter Darstellung,

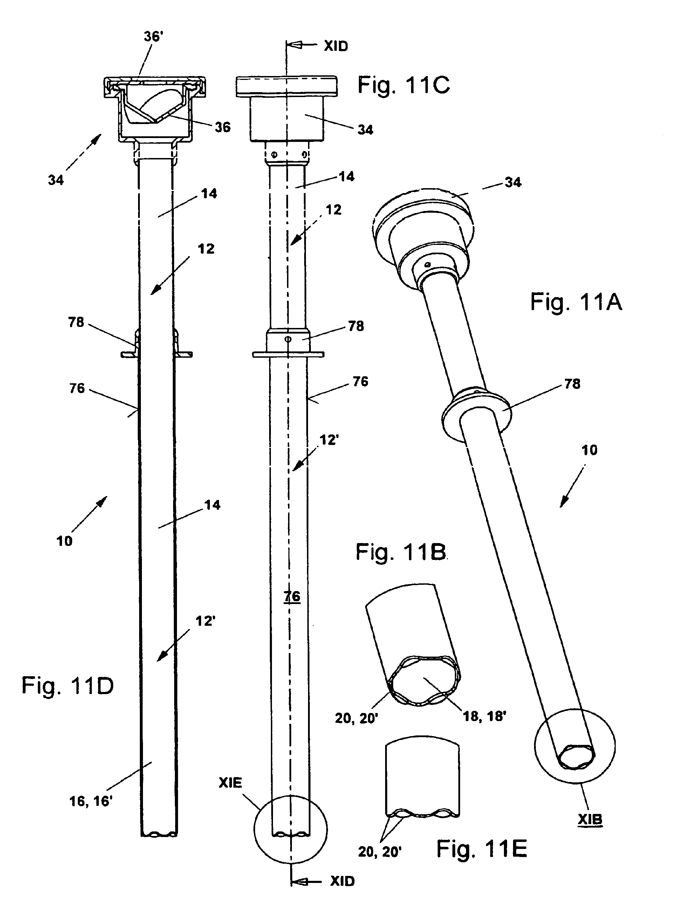

- Fig. 11A bis 11D: eine perspektivische Ansicht, eine end- seitig vergrößerte, teilweise abgebrochene perspektivi- sche Vorderansicht entsprechend Ausschnitt XIB in der Fig. 11A, eine Seitenansicht, eine Querschnittansicht entsprechend der Linie XID-XID in der Fig. 11C und eine endseitig vergrößerte, teilweise abgebrochene Seitenan- sicht entsprechend Ausschnitt XIE in der Fig. 11C einer anderen Ausführungsform einer erfindungsgemäßen Vor- richtung,

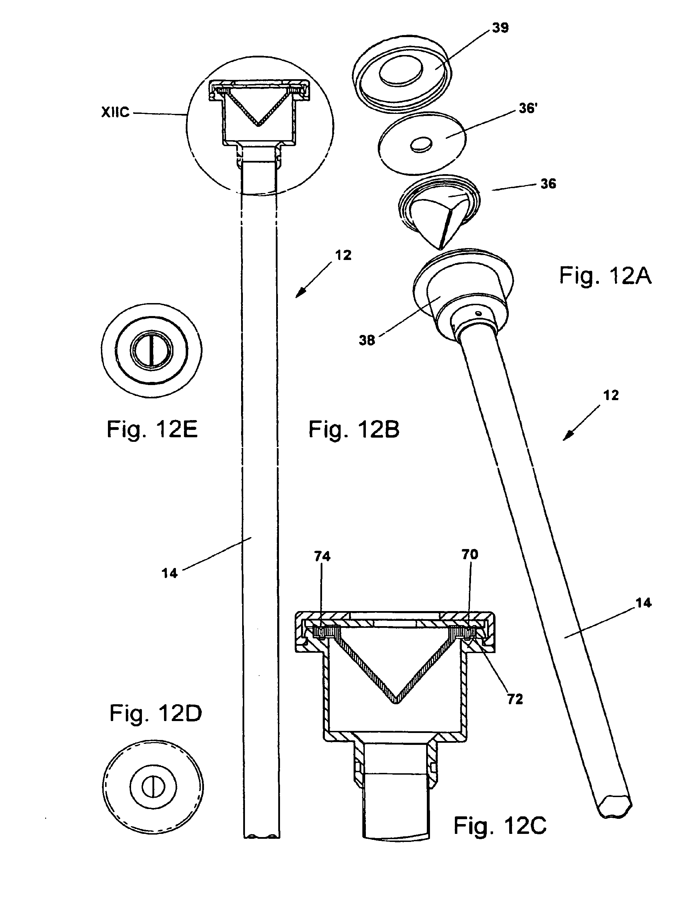

- Fig. 12A bis 12E: eine auseinandergezogene, perspektivische Vorderansicht, eine teilweise aufgebrochene Seitenan- sicht, eine endseitig vergrößerte, teilweise abgebro- chene Querschnittansicht entsprechend Ausschnitt XIIC in der Fig. 12B, eine Vorderansicht und eine Rückan- sicht einer inneren Schneideeinrichtung der anderen Ausführungsform der erfindungsgemäßen Vorrichtung nach den Fig. 11A bis 11E,

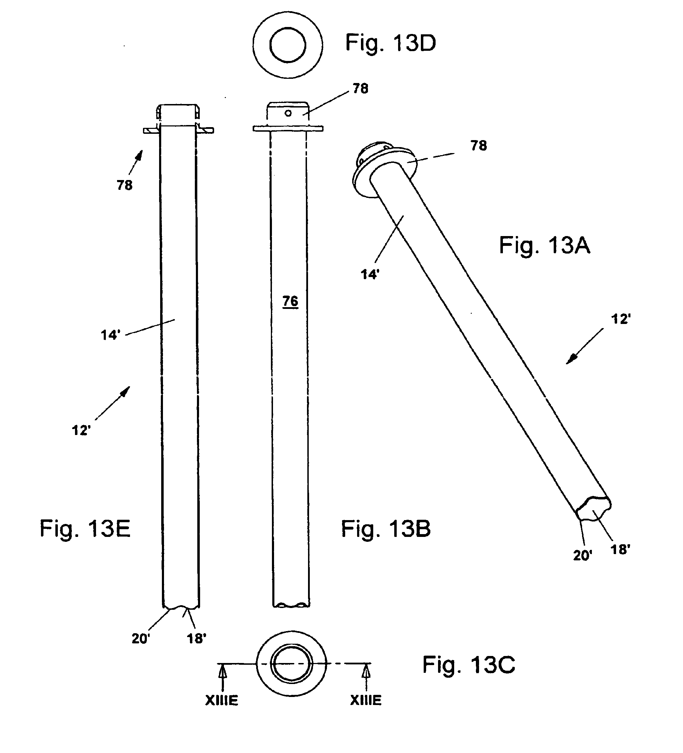

- Fig. 13A bis 13E: eine perspektivische Vorderansicht, eine Seitenansicht, eine Vorderansicht, eine Rückansicht und eine Querschnittansicht entsprechend Linie XIIIE-XIIIE in der Fig. 13C durch eine äußere Schneideeinrichtung der anderen Ausführungsform der erfindungsgemäßen Vor- richtung nach den Fig. 11A bis 11E,

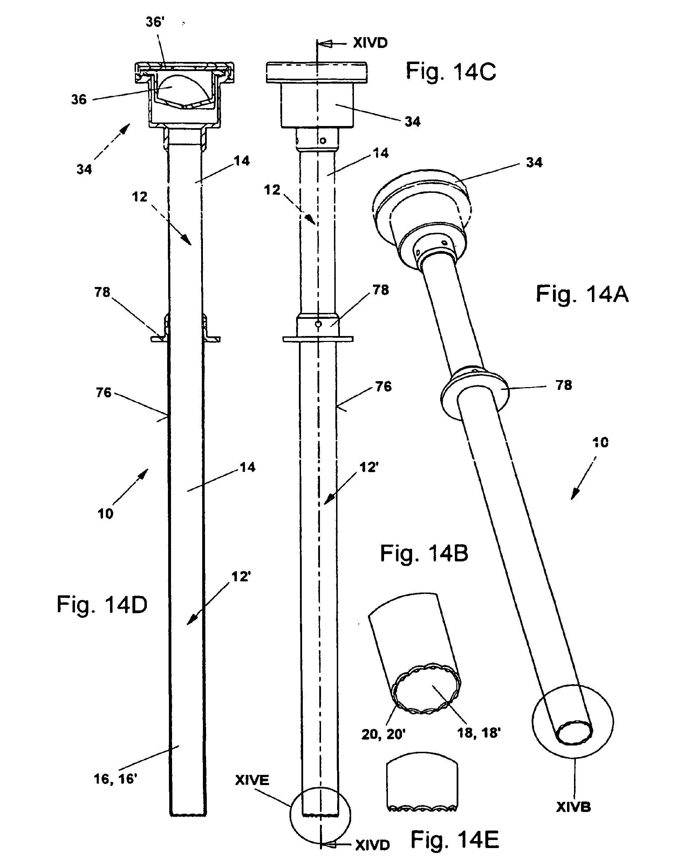

- Fig. 14A bis 14D: eine perspektivische Ansicht, eine end- seitig vergrößerte, teilweise abgebrochene perspektivi- sche Vorderansicht entsprechend Ausschnitt XIVB in der Fig. 14A, eine Seitenansicht, eine Querschnittansicht entsprechend der Linie XIVD-XIVD in der Fig. 14C und eine endseitig vergrößerte, teilweise abgebrochene Sei- tenansicht entsprechend Ausschnitt XIVE in der Fig. 14C einer noch anderen Ausführungsform einer erfindungsge- mäßen Vorrichtung,

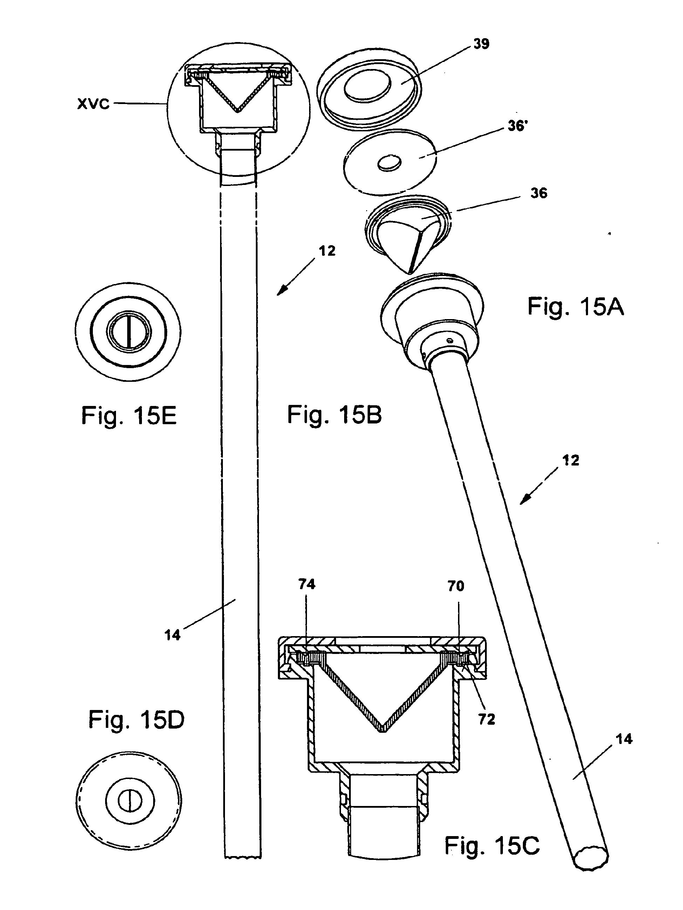

- Fig. 15A bis 15E: eine auseinandergezogene, perspektivische Vorderansicht, eine teilweise aufgebrochene Seitenan- sicht, eine endseitig vergrößerte, teilweise abgebro- chene Querschnittansicht entsprechend Ausschnitt XVC in der Fig. 15B, eine Vorderansicht und eine Rückansicht einer inneren Schneideeinrichtung der anderen Ausfüh- rungsform der erfindungsgemäßen Vorrichtung nach den Fig. 15A bis 15E, und

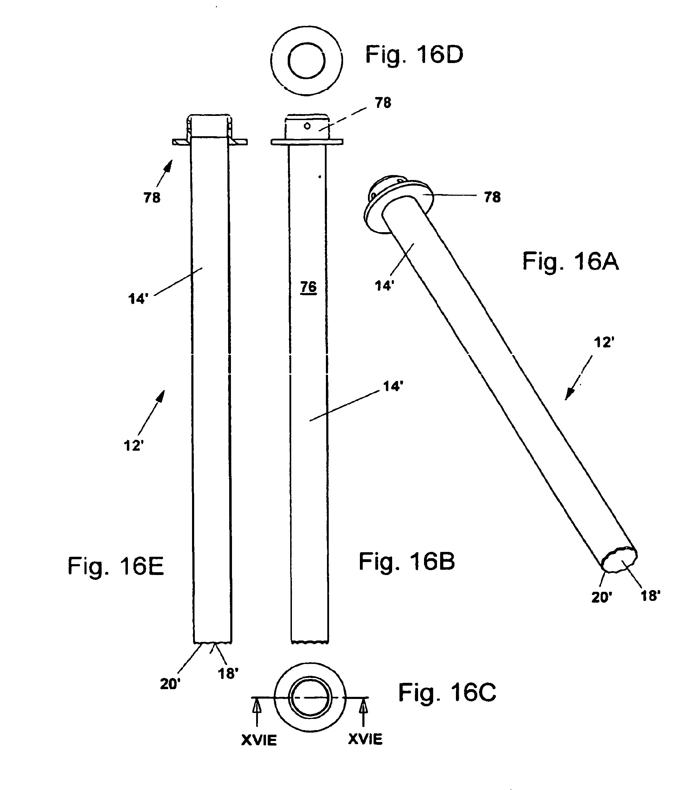

- Fig. 16A bis 16E: eine perspektivische Vorderansicht, eine Seitenansicht, eine Vorderansicht, eine Rückansicht und eine Querschnittansicht entsprechend Linie XVIE-XVIE in der Fig. 16C durch eine äußere Schneideeinrichtung der anderen Ausführungsform der erfindungsgemäßen Vorrich- tung nach den Fig. 16A bis 16E.

Die erfindungsgemäße Vorrichtung 10 ist zum Ausschneiden und Entfernen von Gewebezylindern aus einem Gewebe, das sich innerhalb einer Körper- oder Gelenkhöhle und/oder in bzw. an einem Wandbereich davon befindet, vorgesehen. Bei der nachfolgenden Beschreibung verschiedener Ausführungsbeispiele der erfindungsgemäßen Vorrichtung 10 sind einander entsprechende, gleiche Bauteile jeweils mit identischen Bezugsziffern versehen.

Die Vorrichtung 10 nach der Erfindung eignet sich im weitesten Sinne zum Operieren, insbesondere Entfernen, eines organischen Gewebes, vorzugsweise von Myomen, Geschwülsten, Geschwüren, Karzinomen etc., oder eines anorganischen Körpers, wie Gallen- und Blasensteinen oder dergleichen Agglomerationen, die von einer Körper- oder Gelenkhöhle bzw. einem organischen Hohlraum eines menschlichen bzw. tierischen Körpers und/oder in bzw. an einem Wandbereich davon wenigstens teilweise aufgenommen und/oder umgeben sind.

In den Fig. 1 bis 5 ist nun eine erste Ausführungsform einer solchen Vorrichtung 10 nach der Erfindung dargestellt.

Die Vorrichtung 10 umfasst eine Schneideeinrichtung 12 mit einem hohlzylindrischen Grundkörper 14. An dem distalen Ende 16 des Grundkörpers 14 befindet sich eine distale Öffnung 18. Die distale Öffnung 18 ist von Schneideelement 20 umgeben.

Des Weiteren ist die Vorrichtung 10 mit einer Betätigungseinrichtung 22 versehen, welche zum Drehen des Grundkörpers 14 um dessen Längsachse 24 vorgesehen ist. Die Betätigungseinrichtung 22 ist an einem proximalen Ende 26 des Grundkörpers 14 angeordnet. Alternativ oder - wie bei dem vorliegenden Ausführungsbeispiel gezeigt ist - kumulativ kann die Betätigungseinrichtung 22 auch an einem Bereich 28 des Grundkörpers 14 angeordnet sein, welcher dem proximalen Ende 26 lediglich zugewandt und/oder benachbart ist.

Darüber hinaus weist die Vorrichtung 10 eine Dichtungseinrichtung 30 zum Verschließen und Freigeben einer proximalen Öffnung 32 auf, die sich an dem proximalen Ende 26 des Grundkörpers 14 befindet. Die Dichtungseinrichtung 30 umfasst ein Gehäuse 34 und wenigstens ein in dem Gehäuse 34 aufnehmbares Dichtungselement 36, 36'. Wie aus den Fig. 1A bis 5 ersichtlich ist, sind bei der gezeigten Ausführungsform der erfindungsgemäßen Vorrichtung 10 insgesamt zwei Dichtungselemente 36, 36' vorgesehen. Ohne im Einzelnen dargestellt zu sein, ist es ohne weiteres denkbar, lediglich ein einziges Dichtungselement, zum Beispiel als integrales Bauteil der beiden gezeigten Dichtungselemente 36, 36', oder ebenso mehr als zwei solcher Dichtungselemente 36, 36' vorzusehen.

Das Gehäuse 34 ist aus einem Gehäuseelement 38 und einem Verschlusselement 39 gebildet.

Das Gehäuseelement 38 ist im Wesentlichen topfförmig ausgebildet, nimmt das wenigstens eine Dichtungselement 36, 36' auf und ist an dem Grundkörper 14 unlösbar angebracht. Das wenigstens eine Dichtungselement 36, 36' erstreckt sich zumindest zum Teil in einer Kammer 40, die von dem Gehäuseelement 38 des Gehäuses 34 eingeschlossen und somit gebildet ist.

Das Verschlusselement 39 ist deckel- oder kappenförmig ausgestaltet. Das Verschlusselement 39 ist mit einer mittigen, etwa kreisförmig ausgestalteten Bohrung 41 oder dergleichen Ausnehmung versehen. Durch die Bohrung 41 ist ein weiteres Instrument (nicht gezeigt), insbesondere ein Greif- oder sonstiges Schneideinstrument, vorzugsweise ein Krallengeifer oder ein Myombohrer, hindurchführbar, nämlich über die Bohrung 41 durch die proximale Öffnung 32 des Grundkörpers 14, durch die Dichtungseinrichtung 30 am proximalen Ende 26 des Grundkörpers 14, durch den Grundkörper 14 selbst hin zu dem distalen Ende 16 des Grundkörpers 14, durch die distale Öffnung 18 schließlich zu dem zu entfernenden Gewebe(-zylinder) in dem Operationsgebiet. Das Verschlusselement 39 ist wiederum an dem Gehäuseelement 38 befestigbar.

Zu diesem Zweck können das Gehäuseelement 38 und das Verschlusselement 39 des Gehäuses 34 über eine Gewindeverbindung 42 miteinander verbunden werden, und zwar entweder lösbar oder unlösbar. Die konstruktive Ausgestaltung einer lösbaren oder einer unlösbaren Verbindung zwischen Gehäuseelement 38 und Verschlusselement 39 ist abhängig von der Verwendungs- und Bestimmungsart der Vorrichtung 10 in Form eines Einmal- oder Mehrfach-Gebrauchs, der Ausbildung der Vorrichtung 10 bzw. der Auswahl der Materialien, aus welchen die einzelnen Komponenten der erfindungsgemäßen Vorrichtung 10 bestehen, den Wünschen der Anwender usw.

Bei einem Einmal-Gebrauch der Vorrichtung 10 sind das Gehäuseelement 38 und das Verschlusselement 39 über ein Gewinde unter Zwischenanordnung eines Klebemittels, vorzugsweise eines Silikonklebers, miteinander unlösbar verbunden. Auf diese Weise sind eine thermische oder chemische Sterilisation de Vorrichtung 10 und jeder nochmalige bzw. weitere Gebrauch der Vorrichtung 10 ausgeschlossen.

Die Dichtungseinrichtung 30 umfasst, wie bereits erwähnt, bei der Ausführung der Fig. 1A bis 5 zwei Dichtungselemente 36, 36', die in dem Gehäuse 34 auf der Längsachse 24 des Grundkörpers 14 der Schneideeinrichtung 12 zueinander benachbart angeordnet sind und miteinander zusammenwirken.

Eines 36 der zwei Dichtungselemente 36, 36' der Dichtungseinrichtung 30 ist im Wesentlichen kegel-, kegelstumpf-, topf-, hut- oder dergleichen -förmig ausgebildet. Das Dichtungselement 36 umfasst eine Schlitzöffnung 44, die in einer Ebene 46 (senkrecht zur Blattebene), welche gegenüber einer durch die Grundfläche 48 des Dichtungselementes 36 aufgespannten Ebene 50 (senkrecht zur Blattebene) in Richtung des distalen Endes 16 des Grundkörpers 14 versetzt angeordnet ist. Das Dichtungselement 36 erstreckt sich daher mit dem im Wesentlichen kegel-, kegelstumpf-, topf-, hut- oder dergleichen -förmig ausgebildeten Teil bzw. Abschnitt in Richtung des distalen Endes 16 des Grundkörpers 14 und ragt in die Kammer 40 des Gehäuses 34. Zudem weist das Dichtungselement 36 einen sich radial nach außen erstreckenden Umfangsabschnitt 52 auf, der in der durch die Grundfläche 48 des Dichtungselementes 36 aufgespannten Ebene 50 liegt und einer Fixierung in dem Gehäuse 34 dient.

Das Dichtungselement 36 weist wenigstens zwei Dichtlippen 54, 54' auf, die zum Verschließen und Freigeben der Schlitzöffnung 44 in gegenseitigem Wirkeingriff stehen. Aufgrund der Formgebung und Anordnung der zwei Dichtlippen 54, 54' des Dichtungselementes 36 in dem Gehäuse 34 in entgegengesetzter Richtung zu der Wirkung eines innerhalb einer Körper- oder Gelenkhöhle mittels zum Beispiel durch CO₂-Insufflation künstlich aufgebauten Überdruckes, schmiegen sich die Dichtlippen 54, 54' an ein weiteres Instrument (nicht gezeigt), zum Beispiel ein Greif- oder sonstiges Schneideinstrument, insbesondere ein Krallengeifer oder ein Myombohrer, außenseitig an, und zwar unter vollständiger Abdichtung. Auf diese Weise findet quasi eine Verkeilung der Dichtlippen 54, 54' statt. Die Dichtlippen 54, 54' werden an das weitere Instrument angedrückt und verbleiben in dieser Stellung, bis der Überdruck geregelt aufgehoben wird. Somit ist gewährleistet, dass der Überdruck, welcher zum Operieren, d.h. Ausschneiden bzw. Ausstanzen eines Gewebezylinders aus einem Gewebe und beim anschließenden Entfernen des ausgeschnittenen bzw. ausgestanzten Gewebezylinders aus dem Operationsgebiet notwendig ist, dauerhaft aufrechterhalten bleibt. Ein Entweichen von Gas, wie CO₂ oder dergleichen, aus der Körper- oder Gelenkhöhle des Operationsgebietes ist wirksam verhindert.

Ein anderes 36' der zwei Dichtungselemente 36, 36' der Dichtungseinrichtung 30 ist scheibenförmig ausgebildet und ist mit einer mittigen, etwa kreisförmigen Ausnehmung 56 versehen. Durch die Ausnehmung 56 des Dichtungselementes 36' ist das bereits erwähnte, weitere Instrument (nicht gezeigt), insbesondere ein Greif- oder sonstiges Schneideinstrument, vorzugsweise ein Krallengeifer oder ein Myombohrer, hindurchführbar. Die mittige, etwa kreisförmige Ausnehmung 56 ist mit einem Innendurchmesser, welcher kleiner oder gleich einem Außendurchmesser des weiteren Instrumentes ist, versehen.

Wie aus den Fig. 2, 3D und 4 ersichtlich ist, ist das andere Dichtungselement 36' in dem Gehäuse 34 auf der Längsachse 24 des Grundkörpers 14 zwischen dem proximalen Ende 26 des Grundkörpers 14 und dem einen Dichtungselement 36 angeordnet. Insbesondere ist das andere Dichtungselement 36' der Ebene 50, welche durch die Grundfläche 48 des einen Dichtungselementes 36 aufgespannt ist, (unmittelbar) benachbart zugeordnet.

Die zwei Dichtungselemente 36, 36' der Dichtungseinrichtung 30 sind durch eine Klemmscheibe 58 oder dergleichen in dem Gehäuse 34 zueinander fixierbar.

Entsprechend den Fig. 1A bis 3A, 3D und insbesondere 5 ist bei der Ausführung der Vorrichtung 10 das Schneideelement 20 der Schneideeinrichtung 30 im Wesentlichen angefast, kegelförmig, konisch oder schräg von der distalen Öffnung 18 abgewandt nach außen verlaufend ausgebildet. Dabei kann das Schneideelement 20 einen umlaufenden oder diskontinuierlichen Glattschliff, Wellenschliff oder Zahnschliff aufweisen.

Bei der in den Fig. 1A bis 3A, 3D und 5 dargestellten Ausführungsform der Vorrichtung 10 ist das Schneideelement 20 mit einem Wellenschliff versehen, der über den Umfang verteilte Kreisbogenabschnitte mit axial zurücktretenden Wellentälern und axial hervortretenden Wellenbergen aufweist.

Wie aus den Fig. 1A bis 3A weiter zu entnehmen ist, sind die Betätigungseinrichtung 22 und die Dichtungseinrichtung 30 in vorteilhafter Weise integral ausgebildet.

Dabei ist das Gehäuse 34 der Dichtungseinrichtung 30 und insbesondere das Verschlusselement 39 des Gehäuses 34, an dessen Umfang mit einem Profil 60 zum manuellen Betätigen und Drehen des Grundkörpers 14 versehen.

In alternativer und/oder kumulativer Ausgestaltung kann das Gehäuse 34 der Dichtungseinrichtung 30 an dessen Umfang mit einem Profil zum Zusammenwirken mit einem korrespondierenden Gegenprofil eines Motors oder einer Bauteilkomponente davon (jeweils nicht gezeigt) zum automatischen Betätigen und Drehen des Grundkörpers 34 versehen sein. In zweckmäßiger Weise eignen sich dafür Zahnräder, ein Zahnkranz oder ein Zahnring, etc. Bei der in den Fig. 1A bis 5 vorgestellten Ausführungsform ist das Gehäuse 34 zum Beispiel an dessen Umfang mit radial angeordneten Bohrungen 62 oder Ausnehmungen bzw. Vorsprüngen (nicht gezeigt) versehen, die mit korrespondierend ausgebildeten Vorsprüngen bzw. Bohrungen oder Ausnehmungen an dem Motor oder an einer Bauteilkomponente, zum Beispiel einem Getriebe, des Motors in Wirkeingriff stehen.

Schließlich ist die Ausführungsform der Vorrichtung 10, die in den Fig. 1A bis 5 dargestellt ist, noch mit wenigstens einem Zentrier- und/oder Führungselement 64 versehen. Das Zentrier- und/oder Führungselement 64 ist an bzw. auf dem Grundkörper 14 in einem Abstand zu der Dichtungseinrichtung 30 angeordnet. Der Außendurchmesser des Zentrier- und/oder Führungselementes 64, ist größer als der Außendurchmesser des Grundkörpers 14 und entspricht etwa dem Innendurchmesser einer nicht dargestellten Trokarhülse, in welcher der Grundkörper 34 drehbar und hin- sowie herverschiebbar aufgenommen ist.

Die in den Fig. 6 bis 9 dargestellte weitere Ausführungsform der erfindungsgemäßen Vorrichtung 10 stimmt mit derjenigen der Fig. 1A bis 5 weitgehend überein.

Die Ausführungsform der Vorrichtung 10 nach den Fig. 6 bis 9 unterscheidet sich von der zuvor beschriebenen Ausführungsform lediglich dadurch, dass der Durchmesser des Grundkörpers 14 größer gewählt ist. So kann der Grundkörper 14 der Vorrichtung 10 verschiedene Durchmesser, zum Beispiel von 10 mm bis 30 mm, und eine Länge von vorzugsweise 30 cm aufweisen. Wenn der Außendurchmesser des Grundkörpers 14 etwa dem Innendurchmesser der Trokarhülse entspricht, benötigt die Vorrichtung 10 insofern kein Zentrier- und/oder Führungselement 64.

Grundsätzlicher Unterschied besteht dagegen in der Ausgestaltung des Schneideelementes 20 der Schneideeinrichtung 30, das mit einem Zahnschliff versehen ist. Demnach weist das Schneideelement 20 über den Umfang verteilte Zähne mit axial zurücktretenden Zahngründen und axial hervortretenden Zahnspitzen auf.

Schließlich ist in den Fig. 10A und 10B noch eine andere Ausführungsform einer erfindungsgemäßen Dichtungseinrichtung 30 gezeigt. Demnach kann anstelle einer Gewindeverbindung auch eine Rast- oder Schnappverbindung 66 zur gegenseitigen Befestigung von Gehäuseelement 38 und Verschlusselement 39 vorgesehen sein. Dabei sind das Gehäuseelement 38 und das Verschlusselement 39 über wenigstens einen Rastvorsprung 68 oder dergleichen Rastnase an dem Gehäuseelement 38 bzw. an dem Verschlusselement 39 und mindestens einen weiteren Rastvorsprung 68' an dem Verschlusselement 39 bzw. an dem Gehäuseelement 38 miteinander verbunden. Die beiden Rastvorsprünge 68, 68' oder dergleichen Rastnasen wirken zusammen und sind in gegenseitigen Eingriff bringbar.

Vorzugsweise kann/können der wenigstens eine Rastvorsprung 68 oder dergleichen Rastnase und/oder der mindestens eine weitere Rastvorsprung 68' oder dergleichen Rastnase in diesem Zusammenhang derart ausgebildet sein, dass das Gehäuseelement 38 und das Verschlusselement 39 des Gehäuses 34 nach Ineingriffbringung miteinander unlösbar verbunden sind oder nach anschließender Außereingriffbringung miteinander unverbindbar sind. Beispielsweise kann/können einer oder beide der Rastvorsprünge 68, 68' oder dergleichen Rastnase/n mit einer Sollbruchstelle versehen sein, um die Vorrichtung 10 nach einer Außereingriffbringung dauerhaft zu beschädigen und damit für eine erneute Verwendung unbrauchbar zu machen.

Das wenigstens eine, insbesondere die zwei, Dichtungselement/e 36, 36' der Dichtungseinrichtung 30 ist/sind aus flexiblen Kunststoff, insbesondere aus Silikon, gebildet.

Zurückkommend auf die Ausführungsform der erfindungsgemäßen Vorrichtung der Fig. 4, weist das eine Dichtungselement 36 der zwei Dichtungselemente 36, 36' der Dichtungseinrichtung 30 einen kreisförmigen, umlaufenden Steg 70 auf. Der Steg oder dergleichen Vorsprung ist an dem Umfangsabschnitt 52 des Dichtungselementes 36 angeordnet. Bei der in der Fig. 4 gezeigten Ausführungsform der Vorrichtung 10 ist der Steg 70 integral an dem Umfangsabschnitt 52 angebracht, welcher dem distalen Ende 16 des Grundkörpers 14 zugewandt ist.

Der kreisförmige, umlaufende Steg 70 des einen 36 u der zwei Dichtungselemente 36, 36' ist, wie der Fig. 4 deutlich zu entnehmen ist, im Querschnitt etwa viereckig, insbesondere quadratisch oder rechteckförmig, ausgebildet ist. Alternativ dazu könnte jedoch auch ein anderer Querschnitt, zum Beispiel ein U-förmiger oder V-förmiger Querschnitt, gewählt sein.

Entsprechend der Fig. 4 wirkt der kreisförmige, umlaufende Steg 70 des einen Dichtungselementes 36 mit einer Ausnehmung 72, die an dem Gehäuseelement 38 des Gehäuses 34 angeordnet ist, zusammen. Steg 70 Ausnehmung 72 sind in Form und Abmessung aufeinander abgestimmt, um gegenseitig ineinander form- und/oder kraftschlüssig einzugreifen. Steg 70 und Ausnehmung dienen auf diese Weise als zusätzlich Dichtlippe bzw. Dichtungsbarriere. Gleichzeitig wird hierdurch eine Zentrierung zueinander bzw. gegenüber dem Gehäuseelement 38 des Gehäuses 34 erreicht. Auch wird eine zusätzliche Sicherung des Dichtungselementes 36 gegenüber dem Gehäuseelement 38 erhalten. Ein Verrutschen oder eine sonstige Verschiebung des Dichtungselementes 36 aus der Montagestellung in eine unerwünschte Verformungsstellung, in welcher sich die Dichtwirkung nicht mehr aufrechterhalten lässt, ist somit ausgeschlossen.

Ohne im Einzelnen dargestellt zu sein, kann gleichermaßen eine kinematische Umkehr von Steg 70 und Ausnehmung 72 vorgesehen sein. Bei einer solchen Ausführung ist eine kreisförmige, umlaufende Ausnehmung an dem Umfangsabschnitt 52 angebracht, welcher dem distalen Ende 16 des Grundkörpers 14 zugewandt ist. Demgegenüber wäre an dem Gehäuseelement 38 des Gehäuses 34 ein entsprechend ausgebildeter kreisförmiger, umlaufender Steg angeformt.

Schließlich wäre es ebenso denkbar, ohne ebenfalls im Einzelnen dargestellt zu sein, dass auf die Ausnehmung 72 an dem Gehäuseelement oder einem alternativ vorgesehenen Steg gänzlich verzichtet wird, nachdem das eine Dichtungselement eine höhere Verformbarkeit aufweist als das Gehäuseelement. Gleichwohl lassen sich die zuvor aufgezeigten Vorteile durch eine solche Ausgestaltung, nicht in der Weise erreichen, wie bei dem dargestellten Ausführungsbeispiel nach der Fig. 4.

Weiterhin, wie in der Fig. 4 gezeigt ist, ist das eine Dichtungselement 36 der zwei Dichtungselemente 36, 36' der Dichtungseinrichtung 30 an dessen Umfangsabschnitt 52, welcher dem proximalen Ende 26 des Grundkörpers 16 zugewandt ist, mit einer kreisförmigen, umlaufenden Ausnehmung 74 versehen. Die Ausnehmung 74 ist dabei etwa V-förmig ausgebildet, könnte aber ebenso im Querschnitt viereckig, insbesondere quadratisch oder rechteckförmig, oder U-förmig ausgestaltet sein. Die Funktion der Ausnehmung 74 hinsichtlich Zentrierung, Sicherung des Dichtungselementes 36, Verrutschens oder einer sonstigen Verschiebung des Dichtungselementes 36 aus der Montagestellung in eine unerwünschte Verformungsstellung und der Dichtwirkung, ist dieselbe wie diejenige, welche zuvor im Zusammenhang mit dem Steg 70 erläutert wurde.

Demzufolge kann die Ausnehmung 74 mit einem entsprechend geformten kreisförmigen, umlaufenden Steg (bei diesem Beispiel nicht gezeigt) zusammenwirken, der an dem anderen Dichtungselement 36' der zwei Dichtungselemente 36, 36' der Dichtungseinrichtung 30 angeordnet ist, und zwar an dessen außenseitigen Randbereich, welcher dem distalen Ende 16 des Grundkörpers 14 zugewandt ist.

Alternativ wäre gleichermaßen eine kinematische Umkehr von der Ausnehmung 74 an dem einen Dichtungselement 36 und einem Steg oder auch einer glatten Fläche an dem anderen Dichtungselement 36' möglich.

Ohne wiederum im Einzelnen dargestellt zu sein, wäre es ebenso denkbar, dass das andere Dichtungselement 36' der zwei Dichtungselemente 36, 36' der Dichtungseinrichtung 30 an dessen Umfangsabschnitt, welcher dem proximalen Ende 26 des Grundkörpers 14 zugewandt ist, einen kreisförmigen, umlaufenden Steg und/oder eine kreisförmige, umlaufende Ausnehmung aufweist, um mit einer entsprechenden Ausnehmung bzw. einem entsprechenden Steg an der Klemmscheibe 58 oder direkt am deckel- oder kappenförmigen Verschlusselement 39 des Gehäuses 34 zusammenzuwirken bzw. ineinander einzugreifen.

In den Fig. 11A bis 13E ist eine andere Ausführungsform der erfindungsgemäßen Vorrichtung 10 dargestellt. Die Vorrichtung 10 der Fig. 11A bis 11E unterscheidet sich von derjenigen der vorhergehenden Ausführungsform der Fig. 1A bis 5 im Wesentlichen dadurch, dass zwei Schneideeinrichtungen 12, 12' vorgesehen sind. Demzufolge ist die Schneideeinrichtung 12, wie in den Fig. 12A bis 12E dargestellt, von wenigstens einer weiteren Schneideeinrichtung 12', wie in den Fig. 13A bis 13E gezeigt, umgeben ist. Die beiden Schneideeinrichtungen 12, 12' sind ineinander angeordnet und zueinander relativ sowie jeweils einzeln drehbar ausgebildet.

Die innere Schneideeinrichtung 12 ist von der benachbarten äußeren Schneideeinrichtung 12' der wenigstens zwei Schneideeinrichtungen 12, 12' koaxial und insbesondere mit geringem Spiel aufgenommen ist. Sofern die innere Schneideeinrichtung 12 von der benachbarten äußeren Schneideeinrichtung 12' der wenigstens zwei Schneideeinrichtungen 12, 12' mit größerem Spiel aufgenommen ist, ist/sind wenigstens ein Zentrier- und/oder Führungselement (nicht gezeigt) zwischen den Grundkörpern 14, 14' der inneren Schneideeinrichtung 12 und der benachbarten äußeren Schneideeinrichtung 12' angeordnet.

Die Dichtungseinrichtung 30 zum Verschließen und Freigeben der proximalen Öffnung 32 ist an dem proximalen Ende 26 des Grundkörpers 14 zum Ausschneiden und Entfernen von Gewebezylindern aus einem Gewebe an dem hohlzylindrischen Grundkörper 16 der Schneideeinrichtung 12 angeordnet ist.

Zusätzlich ist der Grundkörper 14' der Schneideeinrichtung 12' an dessen Außenumfang 76 und gleichzeitig an dessen proximalen Ende 26' mit einer Antriebsbuchse 78 oder dergleichen drehfest verbunden.

An den distalen Enden 16, 16' der Grundkörper 14, 14' befindet sich jeweils eine distale Öffnung 18, 18'. Die distale Öffnung 18' ist von einem Schneideelement 20, 20' umgeben, das jeweils mit einem Wellenschliff versehen ist. Alternativ wäre ebenso ein Glattschliff denkbar.

Die Betätigungseinrichtung ist in Form von mindestens einer Antriebseinrichtung (nicht näher dargestellt) zum Drehen des jeweiligen Grundkörpers 16, 16' der wenigstens zwei Schneideeinrichtungen 12, 12 um dessen Längsachse ausgestattet. Die wenigstens zwei Schneideeinrichtungen 12, 12' sind durch die Antriebseinrichtung zueinander im Gegenlauf und/oder im Gleichlauf antreibbar.

Zwischen den wenigstens zwei Schneideeinrichtungen 12, 12' und der Antriebseinrichtung kann ein von einem Gehäuse aufgenommenes Getriebe (ebenfalls nicht dargestellt) zwischengeordnet sein. Die Antriebseinrichtung ist mittels einer, insbesondere elektronischen, Steuer- und/oder Regeleinrichtung steuerbar und/oder regelbar, derart, dass die zwei Schneideinrichtungen 12, 12' durch die Antriebseinrichtung 22 jeweils intervallartig in entgegengesetzter Antriebsrichtung antreibbar sind. Die Zeitintervalle zum Antrieb der zwei Schneideinrichtungen 12, 12' sind in entgegengesetzter Antriebsrichtung durch die, insbesondere elektronische, Steuer- und/oder Regeleinrichtung beliebig einstellbar und/oder voreinstellbar.

Im Übrigen stimmt die Ausführungsform der Fig. 11A bis 13E mit derjenigen nach den Fig. 1A bis 5 überein.

Die Ausführungsform der Vorrichtung 10 nach der Erfindung, die in den Fig. 14A bis 16E gezeigt ist, entspricht etwa derjenigen nach den Fig. 11A bis 13E. Unterschiedlich ist jedoch die Ausbildung der Schneideelemente 20, 20', die nicht als Wellenschliff, sondern als Zahnschliff besitzen, also etwa auch mit der Ausführung nach den Fig. 6 bis 9 kongruent sind.

Das Gehäuseelement 38 und/oder das Verschlusselement 39 des Gehäuses 34 und/oder die Klemmscheibe 58 und/oder das Zentrier- und/oder Führungselement 64 ist/sind aus Kunststoff, insbesondere Polyoximethylen, Polyester, ABS, Acryl, Polycarbonat, Tetrafluorethylen oder Impax, duroplastischen Elastomeren, mit oder ohne Glasfaserverstärkung gebildet. Anstelle dessen kann/können das Gehäuseelement 38 und/oder das Verschlusselement 39 des Gehäuses 34 und/oder die Klemmscheibe 58 und/oder das Zentrier- und/oder Führungselement 64 alternativ und/oder kumulativ aus Metall, insbesondere (nicht-rostendem) Stahl, Edelstahl, Aluminium, Messing, Zink, Rotgusslegierungen oder einer Legierung hieraus gebildet sein. Demgegenüber ist der Grundkörper 14 der Schneideeinrichtung 12 vorzugsweise in aller Regel aus Metall, insbesondere (nicht-rostendem) Stahl, Edelstahl, Aluminium, Messing, Zink, Rotgusslegierungen oder einer Legierung hieraus gebildet.

Die Erfindung ist nicht auf die dargestellten Ausführungsformen der Vorrichtung 10 entsprechend den Fig. 1 bis 10B beschränkt. So ist es möglich, die Rast- oder Schnappverbindung 66 zur gegenseitigen Befestigung von Gehäuseelement 38 und Verschlusselement 39 dahingehend abzuändern, als anstelle eines der beiden Rastvorsprünge 68 bzw. 68' oder dergleichen Rastnase/n eine Rastausnehmung (nicht dargestellt) oder dergleichen vorgesehen ist, in welche der andere der beiden Rastvorsprünge 68' bzw. 68 eingreift und unlösbar, d.h. jedenfalls unter Zerstörung der Rast- oder Schnappverbindung, verrastet.

## Patentansprüche

1. Vorrichtung zum Ausschneiden und Entfernen von Gewebezylindern aus einem Gewebe, das sich innerhalb einer Körper- oder Gelenkhöhle und/oder in bzw. an einem Wandbereich davon befindet, die eine Schneideeinrichtung (12) mit einem hohlzylindrischen Grundkörper (14), einer distalen Öffnung (18) an einem distalen Ende (16) des Grundkörpers (14) und einem die distale Öffnung (18) umgebenden Schneideelement (20), eine Betätigungseinrichtung (22) zum Drehen des Grundkörpers (14) um dessen Längsachse (24) an einem proximalen Ende (26) und/oder an einem dem proximalen Ende (26) zugewandten Bereich (28) des Grundkörpers (14) und eine Dichtungseinrichtung (30) zum Verschließen und Freigeben einer proximalen Öffnung (32) an dem proximalen Ende (26) des Grundkörpers (14) umfasst, wobei die Dichtungseinrichtung (30) ein Gehäuse (34), das ein im Wesentlichen topfförmig ausgebildetes und an dem Grundkörper (14) angebrachtes Gehäuseelement (38) und ein an dem Gehäuseelement (38) befestigbares deckel- oder kappenförmiges Verschlusselement (39) aufweist, und zwei in dem Gehäuse (34) aufnehmbare Dichtungselemente (36, 36') umfasst, wobei das eine Dichtungselement (36) im Wesentlichen kegel-, kegelstumpf-, topf- oder hutförmig ausgebildet ist und wobei das andere Dichtungselement (36') scheibenförmig ausgebildet ist, **dadurch gekennzeichnet, dass** die Dichtungselemente (36, 36') in dem Gehäuse (34) auf der Längsachse (24) des Grundkörpers (14) der Schneideeinrichtung (12) zueinander benachbart angeordnet sind und miteinander zusammenwirken, wobei das eine Dichtungselement (36) mit einem sich radial nach außen erstreckenden Umfangsabschnitt (52) in der durch die Grundfläche (48) des Dichtungselementes (36) aufgespannten Ebene (50) zum Fixieren in dem Gehäuse (34) versehen ist und wobei das eine Dichtungselement (36) an dessen Umfangsabschnitt (52) einen kreisförmigen, umlaufenden Steg (70) oder eine kreisförmige, umlaufende Ausnehmung und das Gehäuseelement (38) eine/n dem Steg (70) oder der Ausnehmung zugewandte/n, entsprechend ausgebildete/n Ausnehmung (72) oder Steg, die zum gegenseitigen Eingriff in Form und Abmessung aufeinander ausgebildet sind, aufweisen und/oder wobei das andere Dichtungselement (36') an dessen außenseitigen Randbereich einen kreisförmigen, umlaufenden Steg oder eine kreisförmige, umlaufende Ausnehmung (74) und das eine Dichtungselement (36) eine/n dem Steg (70) oder der Ausnehmung zugewandte/n, entsprechend ausgebildete/n Ausnehmung oder Steg, die zum gegenseitigen Eingriff in Form und Abmessung aufeinander ausgebildet sind, aufweisen und/oder wobei das andere Dichtungselement (36') an dessen außenseitigen Randbereich einen kreisförmigen, umlaufenden Steg oder eine kreisförmige, umlaufende Ausnehmung und das deckel- oder kappenförmige Verschlusselement (39) des Gehäuses (34) eine/n dem Steg oder der Ausnehmung zugewandte/n entsprechend ausgebildete/n Ausnehmung oder Steg, die zum gegenseitigen Eingriff in Form und Abmessung aufeinander ausgebildet sind, aufweisen.

2. Vorrichtung nach Anspruch 1, **dadurch gekennzeichnet, dass** das Gehäuseelement (38) und das Verschlusselement (39) des Gehäuses (39). über eine Gewindeverbindung (42) oder eine Rast- oder Schnappverbindung (66) miteinander lösbar oder unlösbar verbunden sind.

3. Vorrichtung nach Anspruch 1 oder 2, **dadurch gekennzeichnet, dass** das Gehäuseelement (38) und das Verschlusselement (39) des Gehäuses (34) über ein Gewinde unter Zwischenanordnung eines Klebemittels, vorzugsweise eines Silikonklebers, miteinander unlösbar verbunden sind.

4. Vorrichtung nach einem der Ansprüche 1 bis 3, **dadurch gekennzeichnet, dass** das Gehäuseelement (38) und das Verschlusselement (39) des Gehäuses (34) über wenigstens einen Rastvorsprung (68) oder dergleichen Rastnase an dem Gehäuseelement (38) bzw. an dem Verschlusselement (39) und mindestens einen weiteren Rastvorsprung (68') oder dergleichen Rastnase bzw. mindestens eine Rastausnehmung oder dergleichen an dem Verschlusselement (39) bzw. an dem Gehäuseelement (38), die zusammenwirken und in gegenseitigen Eingriff bringbar sind, miteinander verbunden sind.

5. Vorrichtung nach Anspruch 4, **dadurch gekennzeichnet, dass** der wenigstens eine Rastvorsprung (68) oder dergleichen Rastnase an dem Gehäuseelement (38) bzw. an dem Verschlusselement (39) und der mindestens eine weitere Rastvorsprung (68') oder dergleichen Rastnase bzw. die mindestens eine Rastausnehmung oder dergleichen an dem Verschlusselement (39) bzw. an dem Gehäuseelement (38) derart ausgebildet ist/sind, dass das Gehäuseelement (38) und das Verschlusselement (39) des Gehäuses (34) nach Ineingriffbringung von dem wenigstens einen Rastvorsprung (68) oder dergleichen Rastnase und dem mindestens einen weiteren Rastvorsprung (68') oder dergleichen Rastnase bzw. der mindestens einen Rastausnehmung miteinander unlösbar verbunden sind oder nach anschließender Außereingriffbringung von dem wenigstens einen Rastvorsprung (68) oder dergleichen Rastnase und dem mindestens einen weiteren Rastvorsprung (68') oder dergleichen Rastnase bzw. der mindestens einen Rastausnehmung miteinander unverbindbar sind.

6. Vorrichtung nach einem der Ansprüche 1 bis 5, **dadurch gekennzeichnet, dass** das eine (36) der zwei Dichtungselemente (36, 36') der Dichtungseinrichtung (30) eine Schlitzöffnung (44), die in einer Ebene (46), welche gegenüber einer durch die Grundfläche (48) des Dichtungselementes (36) aufgespannten Ebene (50) in Richtung des distalen Endes (16) des Grundkörpers (14) der Schneideeinrichtung (12) versetzt angeordnet ist, umfasst.

7. Vorrichtung nach einem der Ansprüche 1 bis 6, **dadurch gekennzeichnet, dass** das eine (36) der zwei Dichtungselemente (36, 36') wenigstens zwei Dichtlippen (54, 54') aufweist, die zum Verschließen und Freigeben der Schlitzöffnung (44) in gegenseitigem Wirkeingriff stehen.

8. Vorrichtung nach einem der Ansprüche 1 bis 7, **dadurch gekennzeichnet, dass** das andere (36') der zwei Dichtungselemente (36, 36') der Dichtungseinrichtung (30) eine mittige, etwa kreisförmige Ausnehmung (56)zum Hindurchführen eines weiteren Instrumentes, insbesondere eines Greif- oder sonstiges Schneideinstrumentes, vorzugsweise eines Krallengeifers oder eines Myombohrers, aufweist.

9. Vorrichtung nach Anspruch 8, **dadurch gekennzeichnet, dass** die mittige, etwa kreisförmige Ausnehmung (56) des anderen (36') der zwei Dichtungselemente (36, 36') einen Innendurchmesser, welcher kleiner oder gleich einem Außendurchmesser eines weiteren, durch die Ausnehmung (56) hindurchzuführenden Instrumentes, insbesondere eines Greif- oder sonstiges Schneideinstrumentes, vorzugsweise eines Krallengeifers oder eines Myombohrers, ist, aufweist.

10. Vorrichtung nach einem der Ansprüche 1 bis 9, **dadurch gekennzeichnet, dass** das andere (36') der zwei Dichtungselemente (36, 36') in dem Gehäuse (34) auf der Längsachse (24) des Grundkörpers (14) der Schneideeinrichtung (12) zwischen dem proximalen Ende (26) des Grundkörpers (14) und dem einen (36) der zwei Dichtungselemente (36, 36'), insbesondere der durch die Grundfläche (48) des einen (36) der zwei Dichtungselemente (36, 36') aufgespannten Ebene (50) benachbart, angeordnet ist.

11. Vorrichtung nach einem der Ansprüche 1 bis 10, **dadurch gekennzeichnet, dass** der kreisförmige, umlaufende Steg (70) und/oder die kreisförmige, umlaufende Ausnehmung (74) des einen (36) und/oder des anderen (36') der zwei Dichtungselemente (36, 36') der Dichtungseinrichtung (30) im Querschnitt viereckig, insbesondere quadratisch oder rechteckförmig, U-förmig oder V-förmig ausgebildet ist/sind.

12. Vorrichtung nach einem der Ansprüche 1 bis 11, **dadurch gekennzeichnet, dass** die zwei Dichtungselemente (36, 36') der Dichtungseinrichtung (30) durch eine Klemmscheibe (58) oder dergleichen in dem Gehäuse (34) zueinander fixierbar sind.

13. Vorrichtung nach einem der Ansprüche 1 bis 12, **dadurch gekennzeichnet, dass** das wenigstens eine, insbesondere die zwei, Dichtungselement/e (36, 36') der Dichtungseinrichtung (30) aus flexiblen Kunststoff, insbesondere aus Silikon, gebildet ist/sind.

14. Vorrichtung nach einem der Ansprüche 1 bis 13, **dadurch gekennzeichnet, dass** die Dichtungseinrichtung (30) zum Verschließen und Freigeben der proximalen Öffnung (32) an dem proximalen Ende (26) des Grundkörpers (14) zum Ausschneiden und Entfernen von Gewebezylindern aus einem Gewebe an dem hohlzylindrischen Grundkörper (14) der Schneideeinrichtung (12) angeordnet ist, das von wenigstens einer weiteren Schneideeinrichtung (12') umgeben ist, die ineinander angeordnet und zueinander relativ sowie jeweils einzeln drehbar ausgebildet sind, und mindestens eine Antriebseinrichtung zum Drehen des jeweiligen Grundkörpers (14, 14') der wenigstens zwei Schneideeinrichtungen (12, 12') um dessen Längsachse umfasst.

15. Vorrichtung nach Anspruch 14, **dadurch gekennzeichnet, dass** die wenigstens zwei Schneideeinrichtungen (12, 12') durch eine Antriebseinrichtung zueinander im Gegenlauf und/oder im Gleichlauf antreibbar sind.

16. Vorrichtung nach Anspruch 15, **dadurch gekennzeichnet, dass** zwischen den wenigstens zwei Schneideeinrichtungen (12, 12') und der Antriebseinrichtung ein von einem Gehäuse aufgenommenes Getriebe zwischengeordnet ist.

17. Vorrichtung nach Anspruch 15 oder 16, **dadurch gekennzeichnet, dass** die Antriebseinrichtung mittels einer, insbesondere elektronischen, Steuer- und/oder Regeleinrichtung steuerbar und/oder regelbar ist, derart, dass die zwei Schneideinrichtungen (12, 12') durch die Antriebseinrichtung (22) jeweils intervallartig in entgegengesetzter Antriebsrichtung antreibbar sind.

18. Vorrichtung nach einem der Ansprüche 1 bis 17, **dadurch gekennzeichnet, dass** das Schneideelement (20, 20') der Schneideeinrichtung (12, 12') im Wesentlichen angefast, kegelförmig, konisch oder schräg von der distalen Öffnung (18) abgewandt nach außen verlaufend ausgebildet ist, und insbesondere einen umlaufenden oder diskontinuierlichen Glattschliff, Wellenschliff oder Zahnschliff aufweist.

## Claims

1. Device for excising and removing cylinders of tissue from tissue located within a body cavity or articular cavity and/or in a wall area of same, that comprises a cutting device (12) with a hollow cylindrical main body (14), a distal opening (18) at a distal end (16) of the main body (14) and a cutting element (20) surrounding the distal opening (18), an actuating device (22) at a proximal end (26) and/or at an area (28) of the main body (14) facing towards the proximal end (14) for rotating the main body (14) about the longitudinal axis (24) of said main body (14) and a closure device (30) for closing and opening a proximal opening (32) at the proximal end (26) of the main body (14), with the sealing device (30) comprising a housing (34) which has a housing element (38) being essentially pot-shaped and attached to the main body (14) and a cover- or cap-shaped closure element (39) that can be attached to the housing element (38), and two sealing elements (36, 36') which can be housed in the housing (34), with the one sealing element (36) being essentially of cone, truncated cone, pot or hat shape and with the other sealing element (36') being disc shaped, **characterized in that** the sealing elements (36, 36') are arranged adjacent to each other in the housing (34) along the longitudinal axis (24) of the main body (14) of the cutting device (12) and interact with each other, with the one sealing element (36) is provided with a circular section (52) extending radially outwards in the plane (50) extending through the base (48) of the sealing element (36) for fixing in the housing (34), and with the one sealing element (36) having a circular, circumferential web (70) or a circular, circumferential recess on its circumferential section (52) and the housing element (38) having a suitably shaped recess (72) or web facing the web (70) or recess, being designed, for mutual engagement, to match each other with respect to shape and dimensions, and/or with the other sealing element (36') having a circular, circumferential web or circular, circumferential recess (74) on its outer border area and the one sealing element (36) having a suitably shaped recess or web facing the web (70) or recess, being designed, for mutual engagement, to match each other with respect to shape and dimensions, and/or with the other sealing element (36') having a circular, circumferential web or circular, circumferential recess on its outer border area and the cover- or cap-shaped closure element (39) of the housing (34) having a suitably shaped recess or web facing the web or recess, being designed, for mutual engagement, to match each other with respect to shape and dimensions.

2. Device according to Claim 1, **characterized in that** the housing element (38) and the closure element (39) of the housing (34) are releasably or unreleasably connected to each other by a threaded connection (42) or a latching or snap-in connection (66).

3. Device according to Claims 1 or 2, **characterized in that** the housing element (38) and the closure element (39) of the housing (34) are unreleasably connected to each other by a threaded part with an adhesive agent, preferably a silicone adhesive, arranged in between.

4. Device according to one of Claims 1 to 3, **characterized in that** the housing element (38) and the closure element (39) of the housing (34) are connected to each other through at least one latching projection (68) or similar latching lug on the housing element (38) or on the closure element (39) and at least one other latching projection (68') or similar latching lug or at least one latching recess or similar on the closure element (39) and/or on the housing element (38) that can interact with each other and can be brought into mutual engagement.

5. Device according Claim 4, **characterized in that** the at least one latching projection (68) or similar latching lug on the housing element (38) and/or on the closure element (39) and the at least one other latching projection (68') or similar latching lug and/or the at least one latching recess or similar on the closure element (39) and/or on the housing element (38) is/are designed in such a way that the housing element (38) and the closure element (39) of the housing (34) are unreleasably connected to each other after the at least one latching projection (68) or similar latching lug and the at least one other latching projection (68') or similar latching lug and/or the at least one latching recess have been brought into engagement with each other, or **in that** they cannot be connected to each other after the at least one latching projection (68) or similar latching lug and the at least one other latching projection (68') or similar latching lug and/or the at least one latching recess have been disengaged from each other.

6. Device according to one of Claims 1 to 5, **characterized in that** the one (36) of the two sealing elements (36, 36') of the sealing device (30) has a slotted opening (44) that is arranged in a plane (46) which is offset in the direction of the distal end (16) of the main body (14) of the cutting device (12) with respect to a plane (50) extending through the base (48) of the sealing element (36).

7. Device according to one of Claims 1 to 6, **characterized in that** the one (36) of the two sealing elements (36, 36') has at least two sealing lips (54, 54') that are in mutual effective engagement to close and open the slotted opening (44).

8. Device according to one of Claims 1 to 7, **characterized in that** the other (36') of the two sealing elements (36, 36') of the sealing device (30) has a central, approximately circular, opening (56) through which a further instrument, especially a gripping instrument or other cutting instrument, preferably a claw gripper or a myoma drill, can be inserted.

9. Device according Claim 8, **characterized in that** the central, approximately circular, opening (56) of the other (36') of the two sealing elements (36, 36') has an internal diameter that is smaller than or equal to an external diameter of another instrument, especially a gripping instrument or other cutting instrument, preferably a claw gripper or a myoma drill, which can be inserted through the opening (56).

10. Device according to one of Claims 1 to 9, **characterized in that** the other (36') of the two sealing elements (36, 36') is arranged in the housing (34) on the longitudinal axis (24) of the main body (14) of the cutting device (12) between the proximal end (26) of the main body (14) and the one (36) of the two sealing elements (36, 36'), especially adjacent to the plane (50) extending through the base (48) of the one (36) of the two sealing elements (36, 36').

11. Device according to one of Claims 1 to 10, **characterized in that** the circular, circumferential web (70) and/or the circular, circumferential recess (74) of the one (36) and/or the other (36') of the two sealing elements (36, 36') of the sealing device (30) has/have a cross section which is a quadrangle, especially square or rectangular, or is/are U-shaped or V-shaped.

12. Device according to one of Claims 1 to 11, **characterized in that** the two sealing elements (36, 36') of the sealing device (30) can be fixed to each other by a clamping disc (58) or similar in the housing (34).

13. Device according to one of Claims 1 to 12, **characterized in that** the at least one, especially the two, sealing element(s) (36, 36') of the sealing device (30) is/are made of flexible plastic, especially silicon.

14. Device according to one of Claims 1 to 13, **characterized in that** the sealing device (30) for closing and opening the proximal opening (32) is arranged, at the proximal end (26) of the main body (14), on the hollow cylindrical main body (14) of the cutting device (12), for excising and removing cylinders of tissue from tissue, with the proximal opening being surrounded by at least one further cutting device (12') and said cutting devices being arranged within each other and designed to be rotatable relative to each other and also individually, and having at least one drive device for rotating the respective main body (14, 14') of the at least two cutting devices (12, 12') about its longitudinal axis.

15. Device according to Claim 14, **characterized in that** the at least two cutting devices (12, 12') can be driven by a drive device, in an opposite direction and/or in the same direction relative to each other.

16. Device according to Claim 15, **characterized in that** a gear unit fitted in a housing is arranged between the at least two cutting devices (12, 12') and the drive device.

17. Device according to Claim 15 or 16, **characterized in that** the drive device can be controlled (closed-circuit control or open-circuit control) by a, especially electronic, controller in such a way that the two cutting devices (12, 12') can be driven by the drive device (22) at intervals in each case in the opposite drive direction.

18. Device according to one of Claims 1 to 17, **characterized in that** the cutting element (20) of the cutting device (12) is designed as essentially chamfered, tapered, conical or sloping, facing away from the distal opening (18) and running outwards, and in particular the cutting element (20) of the cutting device (12) has a surrounding or discontinuous smooth, wavy or saw-tooth edge.

## Revendications

1. Dispositif pour découper et enlever des cylindres de tissu hors d'un tissu qui se trouve à l'intérieur d'une cavité corporelle ou d'une cavité articulaire et/ou dans ou sur une zone de paroi de cette cavité, qui comprend un dispositif de coupe (12) avec un corps de base cylindrique creux (14), une ouverture distale (18) à une extrémité distale (16) du corps de base (14) et un élément de coupe (20) qui entoure l'ouverture distale (18), un système d'actionnement (22) pour faire tourner le corps de base (14) autour de son axe longitudinal (24) à une extrémité proximale (26) et/ou au niveau d'une zone (28), tournée vers l'extrémité proximale (26), du corps de base (14) et un système d'étanchement (30) pour obturer et dégager une ouverture proximale (32) à l'extrémité proximale (26) du corps de base (14), dans lequel le système d'étanchement (30) comprend un boîtier (34), qui comporte un élément de boîtier (38) réalisé essentiellement en forme de pot et monté sur le corps de base (14) et un élément de fermeture (39) en forme de couvercle ou de capuchon, capable d'être fixé sur l'élément de boîtier (38), et deux éléments d'étanchéité (36, 36) capable d'être reçu dans le boîtier (34), dans lequel l'un premier élément d'étanchéité (36) est réalisé essentiellement en forme de cône, de godet ou de chapeau, et le second élément d'étanchéité (36') est réalisé en forme de disque, **caractérisé en ce que** les éléments d'étanchéité (36, 36') sont agencées au voisinage l'un de l'autre dans le boîtier (34) sur l'axe longitudinal (24) du corps de base (14) du système de (12) et coopère l'un avec l'autre, **en ce que** le premier élément d'étanchéité (36) est pourvu d'un tronçon périphérique (52), s'étendant radialement vers l'extérieur, dans le plan (50) défini par la surface de base (48) de l'élément d'étanchéité (36) en vue de la fixation dans le boîtier (34),et **en ce que** le premier élément d'étanchéité (36) comporte sur son tronçon périphérique (52) une barrette périphérique circulaire (70) ou un évidement périphérique circulaire et l'élément de boîtier (38) comporte un évidement (72) ou une barrette tournée vers la barrette (70) ou vers l'évidement et réalisé en correspondance en vue d'un engagement réciproque l'un dans l'autre avec une forme et des dimensions mutuellement adaptées, et/ou le second élément d'étanchéité (36') comporte au niveau de sa zone de bordure extérieure une barrette périphérique circulaire ou un évidement périphérique circulaire (74) et le premier élément d'étanchéité (36) comporte un évidement ou une barrette tournée vers la barrette (70) ou vers l'évidement et réalisé en correspondance en vue d'un engagement réciproque avec une forme et des dimensions mutuellement adaptées, et/ou dans lequel le second élément d'étanchéité (36') comporte au niveau de sa zone de bordure extérieure une barrette périphérique circulaire ou un évidement périphérique circulaire et l'élément de fermeture en forme de couvercle ou de capuchon (39) du boîtier (34) comporte un évidement ou une barrette tournée vers la barrette ou vers l'évidement et réalisé en correspondance en vue d'un engagement réciproque avec une forme et des dimensions mutuellement adaptées.

2. Dispositif selon la revendication 1, **caractérisé en ce que** l'élément de boîtier (38) et l'élément de fermeture (39) du boîtier (34) sont reliés l'un à l'autre de manière détachable ou indétachable via une liaison à vis (42), une liaison à enclenchement ou une liaison à encliquetage (66).

3. Dispositif selon la revendication 1 ou 2, **caractérisé en ce que** l'élément de boîtier (38) et l'élément de fermeture (39) du boîtier (34) sont reliés de manière indétachable l'un à l'autre via une liaison à vis avec interposition d'une colle, de préférence d'une colle au silicone.

4. Dispositif selon l'une des revendications 1 à 3, **caractérisé en ce que** l'élément de boîtier (38) et l'élément de fermeture (39) du boîtier (34) sont reliés l'un à l'autre via au moins une saillie d'enclenchement (68) ou un ergot d'enclenchement similaire sur l'élément de boîtier (38) ou sur l'élément de fermeture (39) et au moins une autre saillie d'enclenchement (68') ou un ergot d'enclenchement similaire ou encore au moins un évidement d'enclenchement ou similaire sur l'élément de fermeture (39) ou sur l'élément de boîtier (38), qui coopèrent et sont susceptibles d'être amenés en engagement réciproque.

5. Dispositif selon la revendication 4, **caractérisé en ce que** ladite au moins une saillie d'enclenchement (68) ou ergot d'enclenchement similaire sur l'élément de boîtier (38) ou sur l'élément de fermeture (39), et ladite au moins une autre saillie d'enclenchement (68') ou ergot d'enclenchement similaire, ou encore ledit au moins un évidement d'enclenchement ou similaire sur l'élément de fermeture (39) ou sur l'élément de boîtier (38) est/sont réalisé(s) de telle façon que l'élément de boîtier (38) et l'élément de fermeture (39) du boîtier (34) sont reliés ensemble de façons indétachable après avoir amené en engagement ladite au moins une saillie d'enclenchement (68) ou ergot d'enclenchement similaire et ladite au moins une autre saillie d'enclenchement (68') ou ergot d'enclenchement similaire ou encore ledit au moins un évidement d'enclenchement, ou encore ne peuvent plus être reliés ensemble après avoir supprimé ensuite l'engagement de ladite au moins une saillie d'enclenchement (68) ou ergot d'enclenchement similaire et ladite au moins une autre saillie d'enclenchement (68') ou ergot d'enclenchement similaire, ou bien dudit au moins un évidement d'enclenchement.

6. Dispositif selon l'une des revendications 1 à 5, **caractérisé en ce que** l'un (36) des deux éléments d'étanchement (36, 36') du système d'étanchement (30) comporte une ouverture en fente (44), qui est agencée dans un plan (46), décalé par rapport au plan (50) défini par la surface de base (48) de l'élément d'étanchement (36), en direction de l'extrémité distale (16) le corps de base (14) du système de coupe (12).

7. Dispositif selon l'une des revendications 1 à 6, **caractérisé en ce que** l'un (36) des deux éléments d'étanchement (36, 36') comprend au moins deux lèvres d'étanchement (54, 54') qui sont en engagement actif réciproque pour la fermeture et l'ouverture de l'ouverture en fente (44).

8. Dispositif selon l'une des revendications 1 à 7, **caractérisé en ce que** l'autre (36') des deux éléments d'étanchement (36, 36') du système d'étanchement (30) comporte un évidement central (36) de forme approximativement circulaire pour la traversée d'un autre instrument, en particulier d'un instrument de préhension ou un autre instrument de coupe, de préférence un instrument de préhension à griffe ou une perceuse "Myom".

9. Dispositif selon la revendication 8, **caractérisé en ce que** l'évidement central (56) de forme approximativement circulaire de l'autre (36) des deux éléments d'étanchement (36, 36') présente un diamètre intérieur qui est inférieur ou égal à un diamètre extérieur d'un autre instrument passé à travers l'évidement (56), en particulier d'un instrument de préhension ou d'un autre instrument de coupe, de préférence d'un instrument de préhension à griffe ou d'une perceuse "Myom".

10. Dispositif selon l'une des revendications 1 à 9, **caractérisé en ce que** l'autre (36') des deux éléments d'étanchement (36, 36') est agencé dans le boîtier (34) sur l'axe longitudinal (24) du corps de base (14) du système de coupe (12) entre l'extrémité proximale (26) du corps de base (14) et l'un (36) des deux éléments d'étanchement (36, 36'), en particulier au voisinage du plan (50) défini par la surface de base (48) de l'un (36) des deux éléments d'étanchement (36, 36').

11. Dispositif selon l'une des revendications 1 à 10, **caractérisé en ce que** la barrette périphérique circulaire (70) et/ou l'évidement périphérique circulaire (74) de l'un (36) et/ou de l'autre (36') des deux éléments d'étanchement (36, 36') du système d'étanchement (30) est/sont réalisés avec une section transversale en forme quadrangulaire, en particulier carrée ou rectangulaire, en forme de U ou en forme de V.

12. Dispositif selon l'une des revendications 1 à 11, **caractérisé en ce que** les deux éléments d'étanchement (36, 36') du système d'étanchement (30) sont susceptibles d'être fixés l'un à l'autre par un disque pinceur (58) ou similaire dans le boîtier (34).

13. Dispositif selon l'une des revendications 1 à 12, **caractérisé en ce que** l'un au moins et en particulier les deux éléments d'étanchement (36, 36') du système d'étanchement (30) est/sont formé(s) en matière plastique flexible, en particulier en silicone.

14. Dispositif selon l'une des revendications 1 à 13, **caractérisé en ce que** le système d'étanchement (30) destiné à fermer et à ouvrir l'ouverture proximale (32) à l'extrémité proximale (26) du corps de base (14) pour découper et enlever des cylindres de tissu hors d'un tissu est agencé sur le corps de base cylindrique creux (14) du système de coupe (12), qui est entouré par au moins un autre système de coupe (12'), lesquels sont agencés l'un dans l'autre et sont réalisés avec possibilité de rotation aussi bien l'un par rapport à l'autre qu'individuellement, et comprend au moins un moyen d'entraînement pour faire tourner le corps de base respectif (14, 14') desdits au moins deux systèmes de coupe (12, 12') autour de son axe longitudinal.

15. Dispositif selon la revendication 14, **caractérisé en ce que** lesdits au moins deux systèmes de coupe (12, 12') sont susceptibles d'être entraînés par un moyen d'entraînement en sens opposé et/ou dans le même sens l'un par rapport à l'autre.

16. Dispositif selon la revendication 15, **caractérisé en ce qu'**un mécanisme logé dans un boîtier est interposé entre lesdits au moins deux systèmes de coupe (12, 12') et le moyen d'entraînement.

17. Dispositif selon l'une des revendications 15 ou 16, **caractérisé en ce que** le moyen d'entraînement est susceptible d'être commandé et/ou régulé au moyen d'un système de commande et/ou de régulation, en particulier électronique, de telle façon que les deux systèmes de coupe (12, 12') sont susceptibles d'être entraînés par le moyen d'entraînement (22) chacun à intervalles dans une direction d'entraînement opposée.

18. Dispositif selon l'une des revendications 1 à 17, **caractérisé en ce que** l'élément de coupe (20, 20') du système de coupe (12, 12') est réalisé essentiellement biseau, conoïde, conique ou oblique, tourné de l'ouverture distale (18) vers l'extérieur, et en particulier comprend un circulaire ou discontinu tranchant lisse, tranchant des arbres ou tranchant des dentures.
